# EUROPEAN PATENT SPECIFICATION

(11) **EP 0 641 385 B1**
(45) Date of publication and mention of the grant of the patent: **18.12.2002**
(21) Application number: 92909591.7
(22) Date of filing: 25.10.1991
(51) Int. Cl.: C12N 15/12, C12N 5/12, C12P 21/08, A61K 39/395, A61K 49/00, C12N 5/10, C12Q 1/68

(54) **DEVELOPMENT OF DNA PROBES AND IMMUNOLOGICAL REAGENTS OF HUMAN TUMOR ASSOCIATED ANTIGENS**
ENTWICKLUNG VON DNS-SONDEN UND IMMUNOLOGISCHEN REAGENZIEN VON MENSCHLICHEN TUMOR-ASSOZIIERTEN ANTIGENEN
DEVELOPEMENT DE SONDES D'ADN ET REACTIFS IMMUNOLOGIQUES D'ANTIGENES ASSOCIES A UNE TUMEUR D'ORIGINE HUMAINE

(30) Priority: 25.10.1990 US 603804
(43) Date of publication of application: 08.03.1995
(73) Proprietor: The Trustees of Columbia University in the City of New York, New York, NY 10027-6699 (US)
(72) Inventor: FISHER, Paul, B., Scarsdale, NY 10583 (US)
(74) Representative: VOSSIUS & PARTNER
(86) International application number: PCT/US91/07912
(87) International publication number: WO 92/008131

(56) References cited:
- EP-A- 0 346 702
- US-A- 4 631 259
- US-A- 4 634 665
- US-A- 4 681 840
- US-A- 4 699 877
- US-A- 4 786 718
- US-A- 4 871 838
- US-A- 4 892 935
- US-A- 4 914 021
- US-A- 4 935 341
- US-A- 4 994 558
- US-A- 4 996 298
- US-A- 5 028 420
- NATURE., vol.312, 6 December 1984, LONDON GB pages 545 - 548 JEFFREY A. DREBIN ET AL. 'MONOCLONAL ANTIBODIES IDENTIFY A CELL-SURFACE ANTIGEN ASSOCIATED WITH AN ACTIVATED CELLULAR ONCOGENE'
- CANCER RESEARCH, vol.46, May 1986 pages 2482 - 2487 MICHAEL A. HOLLINGSWORTH ET AL. 'ANTIGENS EXPRESSED ON NIH 3T3 CELLS FOLLOWING TRANSFORMATION WITH DNA FROM A HUMAN PANCREATIC TUMOR'

## Description

### Background of the Invention

Throughout this application, various publications are referenced by Arabic numerals within parentheses. Full citations for these publications may be found at the end of the specification immediately preceding the claims.

Studies over the past several years have been based on several hypotheses. (1) Human cancer develops as a consequence of heritable alterations in a cells genotype. These heritable alterations may involve direct changes in the structure and/or expression of specific genes which regulate expression of the transformed state. Genotypic markers and potential inducers of the tumor cell phenotype include the activation of specific oncogenic elements and/or the loss or inactivation of tumor suppressor elements (1,2). (2) The neoplastic phenotype is often characterized by the surface expression of novel tumor associated antigen (TAA) subsets which are specific for different histological types of tumors and which may be differentially expressed in patients with the same tumor histotype. By employing specific cytokines, the level of expression of specific TAAs can be increased resulting in the improved diagnosis, imaging and ultimately enhanced monoclonal antibody (MoAb) based therapy of cancer (3-5). (3) the specific TAA subset expressed in a particular tumor may not be recognized by the patients' own immune system, thereby resulting in the inability of the patient to mount an effective immunological response and destroy the neoplastic lesion. This problem can be overcome by the exogenous application of MoAbs (specifically human MoAbs; primate MoAbs or chimerized murine MoAbs), augmenting the patients' immune potential with cytokines and expression vector-based immunopotentiators and/or modifying a patients' own tumor cells to function as a vaccine. (4) In many cases, the genetic elements(s) which induces the tumorigenic phenotype may also encode for the specific cell surface TAA. It should, therefore, be possible to cotransfer both the tumorigenic phenotype and its corresponding TAA subset to suitable recipient cells by DNA-transfer techniques.

Transformation of NIH3T3 cells with DNA from pancreatic adenocarcinoma cells (HPAF cell line) has been described Antibodies specific for pancreatic adenocarcinoma cell surface antigens have been produced using said transformed NIH3T3 cells (97).

Studies to directly test many of the above hypotheses have been conducted over the past several years and a number of assumptions have now been validated. One initial test case has involved studies on the genetic and immunologic basis of human prostate carcinoma development.

Prostate cancer is currently the most common cancer in adult males and the leading cause of cancer deaths in males over the age of 55 (6). With a prolongation of life span in humans, the incidence of prostatic carcinoma development and acquisition of metastatic potential by prostatic carcinoma cells (specifically to bone) has not been resolved (7). Attempts to demonstrate consistent changes in the expression of known oncogenes (8-10) in prostatic carcinomas has generally proven unsuccessful. Similarly, prior attempts to demonstrate, via calcium mediated DNA-transfection, a dominant focus forming or tumor-inducing gene in either prostatic carcinomas or prostatic carcinoma cell lines have resulted in only limited success (9). Analysis of a single transformed focus which developed in NIH-3T3 cells following transfection with DNA isolated from a prostatic adenocarcinoma indicated the presence of an activated Ki-ras oncogene (9).

These results suggest that the activation of oncogenes in human prostatic carcinomas, at least those detected by DNA-transfection assays in NIH-3T3, is not a frequent event in prostate cancer. However, by employing DNA cotransfection procedures with high molecular weight human prostatic carcinoma DNA (from the LNCaP cell line) and a dominant acting bacterial antibiotic resistance gene followed by tumor induction in nude mice, a tumorigenic phenotype has been successfully transferred to an established rat embryo fibroblast cell line, CREF (14). This putative prostatic carcinoma transforming gene did not result in focus formation in CREF cells in vitro nor was it detected by similar transfection/tumorigenesis approached in NIH-3T3 cells. Tumor-derived CREF transfectants contained human repetitive (Alu) sequences and a common molecular weight Alu fragment was identified in both primary and independent secondary tumor-derived CREF transfectants.

In addition, nude mouse tumor-derived LNCaP DNA transfected CREF cells were also found to express human tumor associated antigens (TAAs) which were used to generate both polyclonal and monoclonal antibodies reactive with the surface of LNCaP cells. These observations support the following hypotheses: (A) LNCaP cells contain a dominant-acting tumor-inducing gene which can be transferred and expressed in CREF, but not NIH-3T3 cells; (B) the presence of common Alu fragment in primary and secondary transfectants suggests that the gene which has been transferred from LNCaP cells to CREF cells is physically linked to this sequence and its presence can be used as a means of isolating and cloning this tumor-inducing genetic element(s); and (C) the presence of TAAs on the surface of primary and secondary tumor-derived CREF transfectants which can be used to generate monoclonal antibodies which interact specifically with LNCaP cells (and not other human tumors, including melanoma and breast carcinoma, normal human skin fibroblasts and CREF cells) further suggests a potential relationship between this putative tumor-inducing prostatic carcinoma gene and expression of the transformed phenotype in LNCaP cells.

In summary, a general method has been developed for identifying genes and producing immunological reagents which encode TAA of human origin. By using the CREF/Transfection/Monoclonal Antibody technology, the inventor has succeeded in transferring gene(s) which may mediate or are associated with human breast carcinoma and glioblastoma multiform and developing both polyclonal and MoAbs recognizing antigens expressed in human breast carcinomas and other carcinomas. The CREF/Transfection/Monoclonal Antibody technology has also been used to identify defined transfected and expressed surface molecules, including the cloned human 170,000 molecular weight (P-glycoprotein) multi-drug resistance gene. CREF and human tumor (breast carcinoma and glioblastoma) cells were transfected with the cloned mdr-1 gene, selected for colchicine resistance and the presence and expression of the mdr-1 gene was demonstrated by Southern and Northern analysis, respectively. These transfected CREF cells were then used to generate MoAbs which react with the P-glycoprotein expressed in transfected human breast and glioblastoma cells expressing an MDR phenotype.

### Summary of the Invention

The present invention provides a method for preparing a hybridoma cell line which produces an antibody which specifically recognizes and binds to a cell surface antigen associated with a neoplastic, human cell. The method comprises: (a) cotransfecting the CREF-Trans 6 cell line (ATCC Accession No. CRL-10584) with DNA isolated from a neoplastic, human cell and DNA encoding a selectable or identifiable trait; (b) selecting transfected cells which express the selectable or identifiable trait; (c) recovering the transfected cells so selected; (d) injecting the transfected cells so recovered into a suitable first murine host; (e) maintaining the resulting first murine host for a period of time effective to induce the injected transfected cells to form a tumor in the first murine host; (f) isolating the resulting tumor from the first murine host; (g) obtaining tumor cells from the tumor so isolated; (h) coating the tumor cells so obtained with an antiserum generated against the CREF-Trans 6 cell line (ATCC Accession No. CRL-10584); (i) injecting the antiserum-coated cells into suitable non-human second hosts; (j) screening the resulting second hosts to identify hosts which produce serum reactive with the neoplastic, human cell; (k) removing spleens from the second hosts so identified; (l) preparing from the spleens so removed hybridomas; and (m) recovering therefrom a hybridoma cell line which produces an antibody which specifically recognizes and binds to the cell surface antigen.

The present invention also provides a method for producing a monoclonal antibody which specifically recognizes and binds to a cell surface antigen associated with a neoplastic, human cell. This method comprises producing a hybridoma according to the above method and recovering from the hybridoma so produced the monoclonal antibody.

The present invention further provides a method for preparing a polyclonal antibody which specifically recognizes and binds to a cell surface antigen associated with a neoplastic, human cell. This method comprises: (a) cotransfecting the CREF-Trans 6 cell line with DNA isolated from a neoplastic, human cell and DNA encoding a selectable or identifiable trait; (b) selecting transfected cells which express the selectable or identifiable trait; (c) recovering the transfected cells so selected; (d) injecting the transfected cells so recovered into a suitable first murine host; (e) maintaining the resulting first murine host for a period of time effective to induce the injected transfected cells to form a tumor in the first murine host; (f) isolating the resulting tumor from the first murine host; (g) obtaining tumor cells from the tumor so isolated; (h) coating the tumor cells so obtained with an antiserum generated against the CREF-Trans 6 cell line; (i) injecting the antiserum-coated cells into suitable non-human second hosts; (j) screening the resulting second hosts to identify hosts which produce serum reactive with the neoplastic, human cell; and (k) recovering from the second hosts so identified the polyclonal antibody.

In addition, the present invention describes a method of diagnosing in a subject a neoplastic condition which comprises contacting a sample from the subject with any of the antibodies above labeled with a detectable marker under conditions permitting the antibody to specifically recognize and bind to the cell surface antigen associated with the neoplastic condition, detecting the presence of antibody bound to the antigen, and thereby diagnosing the neoplastic condition.

The present invention also describes a method of treating a neoplastic condition which comprises contacting neoplastic, human cells associated with the neoplastic condition with any of the antibodies above labeled with a therapeutic agent under conditions such that the therapeutic agent selectively inhibits proliferation of the neoplastic, human cells.

The present invention further describes a method of imaging a neoplastic, human cell which comprises contacting the neoplastic, human cell to be imaged with any of the antibodies above labeled with an imaging agent under conditions permitting the antibody to specifically recognize and bind to the cell surface antigen associated with the neoplastic cell and detecting the imaging agent bound thereto, thereby imaging the neoplastic cell.

The present invention also provides a method for preparing DNA encoding a cell surface antigen associated with a neoplastic, human cell. This method comprises: (a) cotransfecting CREF-Trans 6 cell line with DNA isolated from a neoplastic, human cell and DNA encoding a selectable or identifiable trait; (b) selecting transfected cells which express the selectable or identifiable trait; (c) recovering the transfected cells so selected; (d) injecting the transfected cells so recovered into a suitable first murine host; (e) maintaining the resulting first murine host for a period of time effective to induce the injected transfected cells to form a tumor in the first murine host; (f) isolating the resulting tumor from the first murine host; (g) obtaining tumor cells from the tumor so isolated; and (h) recovering DNA encoding the cell surface antigen associated with the neoplastic, human cell from the tumor cells so obtained.

Lastly, the present invention describes a method of diagnosing in a subject a neoplastic condition which comprises contacting a sample from the subject with the DNA probe labeled with a detectable marker under conditions permitting the DNA probe to hybridize with the DNA associated with the neoplastic condition, detecting the presence of hybridized DNA, and thereby diagnosing the neoplastic condition.

### Brief Description of the Figures

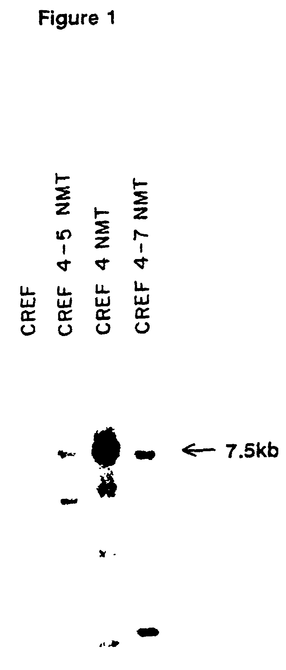

Figure 1. Southern blot analysis. High molecular weight DNA from CREF-Trans 6 (represented as CREF in figure 1), a primary tumor obtained in nude mice following the injection of CREF-Trans 6 cells transfected with pSV2-neo and LNCap DNA (CREF-4 NMT) and two independent secondary tumors obtained following the injection into nude mice of CREF-Trans 6 cells with pSV2-neo and CREF 4 NMT DNA. Southern blots were probed with a 32P-labeled nick-translanted 300 bp Alu probe.

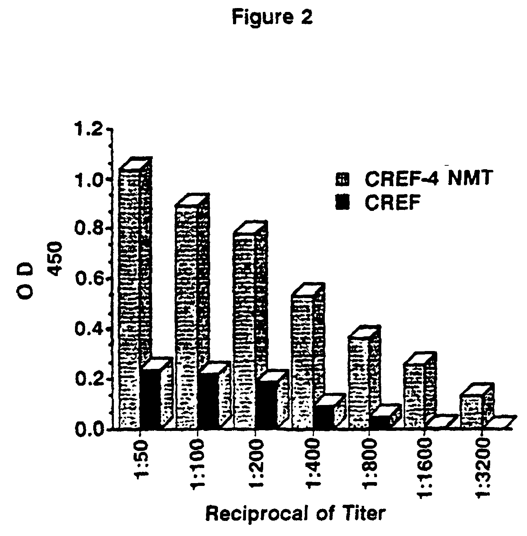

Figure 2. Titration of CREF 4 NMT-coated and CREF-Trans 6 (represented as CREF in figure 2) -coated antisera against LNCaP. Animals immunized with nude mouse tumor-derived CREF-Trans 6 cells transfected with LNCaP DNA (CREF-4 NMT) exhibit an immune response to the original LNCaP cells. CREF-Trans 6 cells lacking the putative human TAAs from LNCaP cells do not generate an immune response to LNCaP cells.

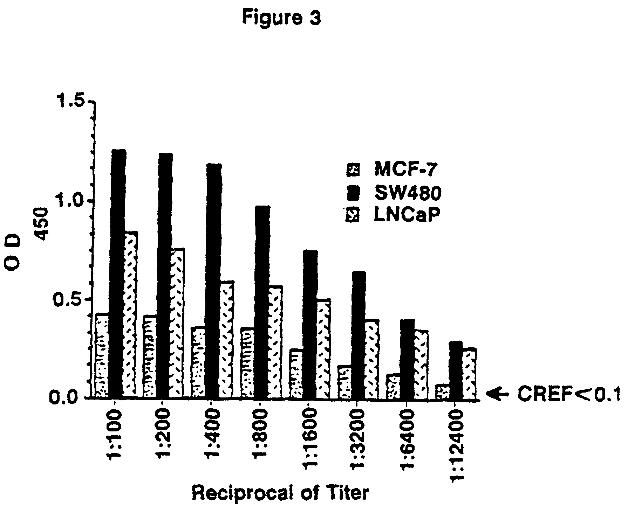

Figure 3. Binding of CREF-4 NMT polyclonal antisera to human carcinoma cells. Polyclonal antibody generated against high titer CREF-Trans 6 cells (represented as CREF cells in figure 3) transfected with LNCaP DNA (CREF-4 NMT) bind to human prostatic, breast and colorectal carcinoma cell lines. In contrast, polyclonal antibody generated against CREF-Trans 6 cells coated with the same high titer CREF-Trans 6 antisera as CREF-4 NMT does not bind to human carcinoma derived cell lines.

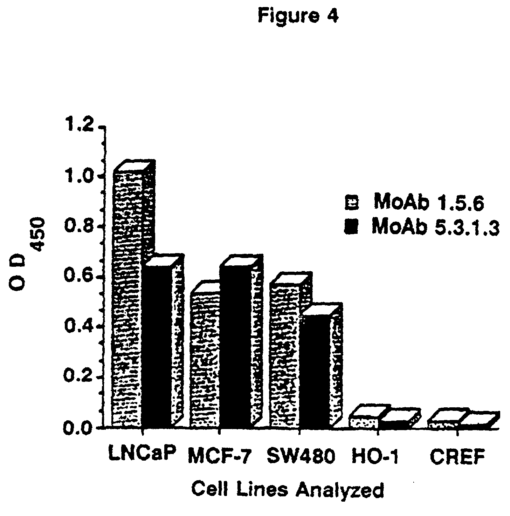

Figure 4. Binding of CREF-4 NMT MoAbs to human tumor cell lines and CREF-Trans 6. MoAbs developed from animals immunized with high-titer CREF-Trans 6 antisera treated CREF-4 NMT cells display good specificity for LNCaP and other human carcinomas. In contrast, the same MoAbs do not bind to HO-I human melanoma, WI-38 human skin fibroblast or CREF-Trans 6 cells (represented as CREF in figure 4).

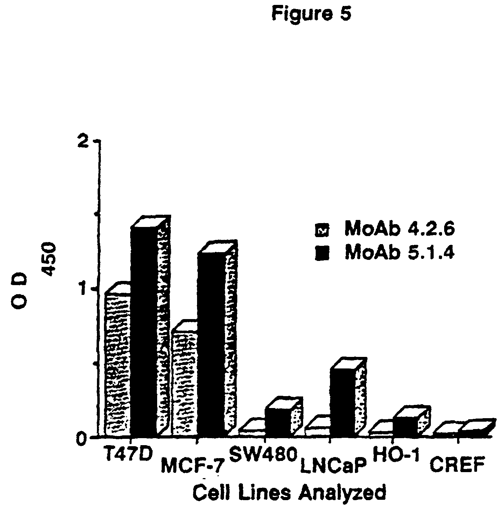

Figure 5. Binding of CREF-T47D MoAbs to human cell lines and CREF-Trans 6 cells. MoAbs generated against nude mouse tumor-derived DNA-transfected CREF-Trans 6 cells exhibit good specificity for human breast carcinoma cells. MoAb 5.1.4 also displays some binding to human prostatic carcinoma cells. MoAbs do not bind to HO-I human melanoma cells or nontransfected CREF-Trans 6 cells (represented as CREF in Figure 5).

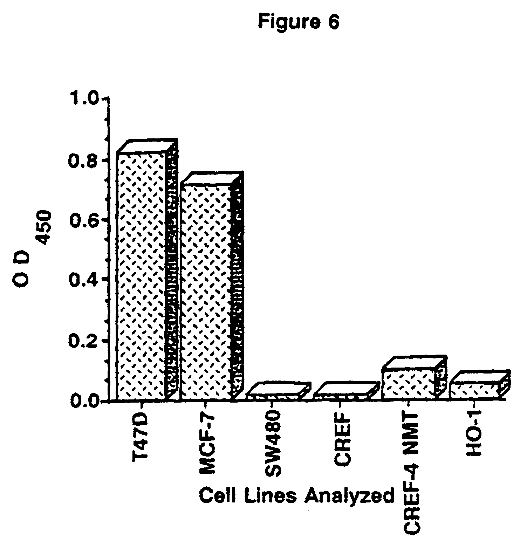

Figure 6. Binding of MoAb 5.1.4 (CREF-T47D) ascites to human and CREF-Trans 6 cell lines (represented as CREF in Figure 6). MoAb 5.1.4 developed against animals immunized with nude mouse tumor-derived CREF-Trans 6 cells transfected with human breast carcinoma DNA from T47D cells (which had been coated prior to injection in animals with high-titer CREF-Trans 6 antisera) continue to display good specificity for human breast carcinoma cells following subcloning and ascites formation.

### Detailed Description of the Invention

The present invention provides a method for preparing a hybridoma cell line which produces an antibody which specifically recognizes and binds to a cell surface antigen associated with a neoplastic, human cell. The method comprises: (a) cotransfecting the CREF-Trans 6 cell line (ATCC Accession No. CRL-10584) with DNA isolated from a neoplastic, human cell and DNA encoding a selectable or identifiable trait; (b) selecting transfected cells which express the selectable or identifiable trait; (c) recovering the transfected cells so selected; (d) injecting the transfected cells so recovered into a suitable first murine host; (e) maintaining the resulting first murine host for a period of time effective to induce the injected transfected cells to form a tumor in the first murine host; (f) isolating the resulting tumor from the first murine host; (g) obtaining tumor cells from the tumor so isolated; (h) coating the tumor cells so obtained with an antiserum generated against the CREF-Trans 6 cell line (ATCC Accession No. CRL-10584); (i) injecting the antiserum-coated cells into suitable second non-human hosts; (j) screening the resulting second hosts to identify hosts which produce serum reactive with the neoplastic, human cell; (k) removing spleens from the second hosts so identified; (l) preparing from the spleens so removed hybridomas; and (m) recovering therefrom a hybridoma cell line which produces an antibody which specifically recognizes and binds to the cell surface antigen.

The CREF-Trans 6 cell line has been deposited with the American Type Culture Collection in Rockville, Maryland 20852, U.S.A., under ATCC Accession No. CRL 10584. This was deposited pursuant to, and in satisfaction of the requirements of the Budapest Treaty on the International Recognition of the Deposit of Microorganisms for the Purposes of Patent Procedure.

The neoplastic, human cell may be any neoplastic, human cell that is benign or metastatic and may be derived from any neoplastic, human cell line or any primary tumor, even very small quantities of primary tumor. In one embodiment of the invention, the neoplastic, human cell is a human prostatic carcinoma cell derived from cell line LNCaP. In another embodiment of the invention, the neoplastic, human cell is a human breast carcinoma cell derived from cell line T47D. In another embodiment of the invention, the neoplastic, human cell is a human colorectal carcinoma cell derived from cell line SW480. In another embodiment of the invention, the neoplastic, human cell is a human glioblastoma multiform (stage IV astrocytoma) cell derived from cell line GBM-18. In yet another embodiment of the invention, the neoplastic, human cell is a human glioblastoma multiform (stage IV astrocytoma) cell derived from a primary tumor.

As used herein, the DNA encoding the selectable or identifiable trait may be any DNA encoding a selectable or identifiable trait. In one embodiment of the invention, the DNA encoding the selectable or identifiable trait is plasmid DNA encoding resistance to an antibiotic. In the preferred embodiment of the invention, the plasmid DNA comprises pSV2-Neo and the antibiotic is G418.

As used herein, the suitable second host can be a murine host or a non-human primate host.

For the purposes of this invention, the cell surface antigen that is associated with a neoplastic, human cell may be any cell surface antigen. The cell surface antigen may be, but is not limited to the following embodiments: a tumor associated antigen, a growth factor receptor, a viral-encoded surface-expressed antigen, an antigen encoded by an oncogene product, a surface epitope, a membrane protein which mediates classical or atypical multi-drug resistance, an antigen which mediates a tumorigenic phenotype, an antigen which mediates a metastatic phenotype, an antigen which suppresses a tumorigenic phenotype, an antigen which suppresses a metastatic phenotype, an antigen which is recognized by a specific immunological effector cell such as a T-cell, and an antigen that is recognized by a non-specific immunological effector cell such as a macrophage cell or a natural killer cell. In the preferred embodiment of the invention, the cell surface antigen is a tumor associated antigen.

The hybridoma cell line can be recovered using methods known to those of ordinary skill in the art.

It is also within the confines of the present invention that steps (a) through (g) may be repeated to obtain additional tumor cells, i.e. secondary transfectants, tertiary transfectants, etc.

The present invention further provides a method for producing a monoclonal antibody which specifically recognizes and binds to a cell surface antigen associated with a neoplastic, human cell. This method comprises producing a hybridoma according to the above method and recovering from the hybridoma so produced the monoclonal antibody.

For purposes of this invention, the monoclonal antibody may be recovered by methods known to those of ordinary skill in the art.

The present invention also provides a method for preparing a polyclonal antibody which specifically recognizes and binds to a cell surface antigen associated with a neoplastic, human cell. This method comprises: (a) cotransfecting the CREF-Trans 6 cell line with DNA isolated from a neoplastic, human cell and DNA encoding a selectable or identifiable trait; (b) selecting transfected cells which express the selectable or identifiable trait; (c) recovering the transfected cells so selected; (d) injecting the transfected cells so recovered into a suitable first murine host; (e) maintaining the resulting first murine host for a period of time effective to induce the injected transfected cells to form a tumor in the first murine host; (f) isolating the resulting tumor from the first murine host; (g) obtaining tumor cells from the tumor so isolated; (h) coating the tumor cells so obtained with an antiserum generated against the CREF-Trans 6 cell line; (i) injecting the antiserum-coated cells into suitable second non-human hosts; (j) screening the resulting second hosts to identify hosts which produce serum reactive with the neoplastic, human cell; and (k) recovering from the second hosts so identified the polyclonal antibody.

For purposes of this invention, the polyclonal antibody can be recovered by methods known to those of ordinary skill in the art.

The neoplastic, human cell may be any neoplastic, human cell that is benign or metastatic and can be derived from any neoplastic, human cell line or any primary tumor, even small quantities of primary tumor. In embodiment of the invention the neoplastic, human cell is a human prostatic carcinoma cell derived from cell line LNCaP. In another embodiment of the invention the neoplastic, human cell is a human breast carcinoma cell derived from cell line T47D. In another embodiment of the invention the neoplastic, human cell is a human colorectal carcinoma cell derived from cell line SW480. In another embodiment of the invention the neoplastic, human cell is a human glioblastoma multiform (stage IV astrocytoma) cell derived from cell line GBM-18. In yet another embodiment of the invention, the neoplastic, human cell is a human glioblastoma multiform (stage IV astrocytoma) cell derived from a primary tumor.

The DNA encoding the selectable or identifiable trait may be any DNA encoding a selectable or identifiable trait. In one embodiment of the invention, the DNA encoding the selectable or identifiable trait is plasmid DNA encoding resistance to an antibiotic. In the preferred embodiment of the invention, the plasmid DNA comprises pSV2-Neo and the antibiotic is G418.

As used herein, the suitable second host can be a murine host or a non-human primate host.

For purposes of the present invention, the cell surface antigen that is associated with a neoplastic, human cell may be any cell surface antigen. The cell surface antigen may be, but is not limited to the following embodiments: a tumor associated antigen, a growth factor receptor, a viral-encoded surface-expressed antigen, an antigen encoded by an oncogene product, a surface epitope, a membrane protein which mediates a classical or atypical multi-drug resistance, an antigen which mediates a tumorigenic phenotype, an antigen which mediates a metastatic phenotype, an antigen which suppresses a tumorigenic phenotype, an antigen which suppresses a metastatic phenotype, an antigen which is recognized by a specific immunological effector cell such as a T-cell, and an antigen that is recognized by a non-specific immunological effector cell such as a macrophage cell or a natural killer cell. In the preferred embodiment of the invention, the cell surface antigen is a tumor associated antigen.

It is within the confines of the present invention that steps (a) through (g) may be repeated to obtain additional tumor cells, i.e. secondary tranfectants, tertiary transfectants, etc.

The present invention also describes the monoclonal or polyclonal antibody above labeled with a detectable marker. As used herein, the detectable markers are well known to those of ordinary skill in the art and may be, but are not limited to enzymes, paramagnetic ions, biotins, fluorophores, chromophores, heavy metals, or radioisotopes.

The present invention also describes a method of diagnosing in a subject a neoplastic condition which comprises contacting a sample from the subject with any of the antibodies above labeled with a detectable marker under conditions permitting the antibody to specifically recognize and bind to the cell surface antigen associated with the neoplastic condition, detecting the presence of antibody bound to the antigen, and thereby diagnosing the neoplastic condition.

The present invention also describes the monoclonal antibody or polyclonal antibody above labeled with a therapeutic agent. As used herein, the therapeutic agents are well known to those of ordinary skill in the art and may be, but are not limited to antibiotics, antiviral agents, such as interferon, toxins, radioisotopes, or chemotherapeutic agents.

The present invention further describes a method of treating a neoplastic condition which comprises contacting neoplastic, human cells associated with the neoplastic condition with any of the antibodies above labeled with a therapeutic agent under conditions such that the therapeutic agent selectively inhibits proliferation of the neoplastic, human cells.

The present invention also describes the monoclonal antibody or polyclonal antibody above labeled with an imaging agent. As used herein, the imaging agents are well known to those or ordinary skill in the art and may be, but are not limited to radioisotopes., dyes or enzymes such as peroxidase or alkaline phosphate, paramagnetic ions, or elements opaque to x-rays.

The present invention also describes a method of imaging a neoplastic, human cell which comprises contacting the neoplastic, human cell to be imaged with any of the antibodies above labeled with an imaging agent under conditions permitting the antibody to specifically recognize and bind to the cell surface antigen associated with the neoplastic cell and detecting the imaging agent bound thereto, thereby imaging the neoplastic cell.

As used herein, the method of imaging may comprise any of the numerous methods of imaging known to those of ordinary skill in the art such as, but not limited to visualizing radiation emitted by a radioactive isotope.

In addition, the present invention provides a method for preparing DNA encoding a cell surface antigen associated with a neoplastic, human cell. This method comprises: (a) cotransfecting the CREF-Trans 6 cell line with DNA isolated from a neoplastic, human cell and DNA encoding a selectable or identifiable trait; (b) selecting transfected cells which express the selectable or identifiable trait; (c) recovering the transfected cells so selected; (d) injecting the transfected cells so recovered into a suitable first murine host; (e) maintaining the resulting first murine host for a period of time effective to induce the injected transfected cells to form a tumor in the first murine host; (f) isolating the resulting tumor from the first murine host; (g) obtaining tumor cells from the tumor so isolated; and (h) recovering DNA encoding the cell surface antigen associated with the neoplastic, human cell from the tumor cells so obtained.

It is within the confines of the present invention that steps (a) through (g) may be repeated to obtain additional tumor cells, i.e. secondary transfectants, tertiary secondary etc.

This method for preparing DNA is an improvement over past methods because this aspect of the invention employs a new cloned rat embryo fibroblast cell line, specifically the CREF-Trans 6 cell line. The CREF-Trans 6 cell line permits the identification of genes mediating a tumor phenotype and cell surface antigen expression using small quantities of tumor tissue. The CREF-Trans 6 cell line also allows for easy detection of human repetitive sequences that serve as a genetic marker delineating the location of genes relevant to the induction of the tumor phenotype and cell surface expression. In addition, the expression of many known oncogenes have not been observed in tumor cells derived from the CREF cell line (21). This argues for the possibility of using this system to identify potentially new classes of human oncogenes.

The method for recovering DNA from the tumor cells so obtained may comprise any of the numerous methods of isolating, purifying and cloning DNA known to one of ordinary skill in the art. This methods may be, but are not limited to polymerase chain reaction or classic phage cloning techniques.

The neoplastic, human cell may be any neoplastic, human cell that is benign or metastatic and can be derived from any neoplastic, human cell line or any primary tumor, even small quantities of primary tumor. In one embodiment of the invention the neoplastic,-human cell is a human prostatic carcinoma cell derived from cell line LNCaP. In another embodiment of the invention the neoplastic, human cell is a human breast carcinoma cell derived from cell line T47D. In another embodiment of the invention the neoplastic, human cell is a human colorectal carcinoma cell derived from cell line SW480. In another embodiment of the invention the neoplastic, human cell is a human glioblastoma multiform (stage IV astrocytoma) cell derived from cell line GBM-18. In yet another embodiment of the invention, the neoplastic, human cell is a human glioblastoma multiform (stage IV astrocytoma) cell derived from a primary tumor.

The DNA encoding the selectable or identifiable trait may be any DNA encoding a selectable or identifiable trait. In one embodiment of the invention, the DNA encoding the selectable or identifiable trait is plasmid DNA encoding resistance to an antibiotic. In the preferred embodiment of the invention, the plasmid DNA comprises pSV2-Neo and the antibiotic is G418.

For purposes of the present invention, the cell surface antigen that is associated with a neoplastic, human cell may be any cell surface antigen. The cell surface antigen may be, but is not limited to the following embodiments: a tumor associated antigen, a growth factor receptor, a viral-encoded surface-expressed antigen, an antigen encoded by an oncogene product, a surface epitope, a membrane protein which mediates classical or atypical multi-drug resistance, an antigen which mediates a tumorigenic phenotype, an antigen which mediates a metastatic phenotype, an antigen which suppresses a tumorigenic phenotype, an antigen which suppresses a metastatic phenotype, an antigen which is recognized by a specific immunological effector cell such as a T-cell, and an antigen that is recognized by a non-specific immunological effector cell such as a macrophage cell or a natural killer cell. In the preferred embodiment of the invention, the cell surface antigen is a tumor associated antigen.

This invention also describes a DNA probe hybridizable with the DNA above. This invention further describes the DNA probe above which is labeled with a detectable marker. As used herein, the detectable markers are well known to those of ordinary skill in the art and may be, but are not limited to enzymes, paramagnetic ions, biotins, fluorophores, chromophores, heavy metals, or radioisotopes.

Lastly, the present invention describes a method of diagnosing in a subject a neoplastic condition which comprises contacting a sample from the subject with the DNA probe labeled with a detectable marker under conditions permitting the DNA probe to hybridize with the DNA associated with the neoplastic condition, detecting the presence of hybridized DNA, and thereby diagnosing the neoplastic condition. The presence of the probe indicates the detection of a neoplastic condition.

The present invention is further illustrated in the Experimental Details section which follows. This section is set forth to aid in an understanding of the invention but is not intended to, and should not be construed to, limit in any way the invention as set forth in the claims which follow.

### Experimental Details

### Materials and Methods:

CREF-Trans 6 Cell Line. The CREF-Trans 6 cell line is a subclone of the CREF cell line which is a specific clone of Fisher F2408 rat embryo cells (33). It was developed by plating CREF cells at low densities (50 and 100 cells/6-cm plate) and isolating a series of single-cell subclones. These subclones of CREF (identified as CREF-Trans 1 through CREF-Trans 20) were then tested for their ability to be morphologically transformed following transfection with the Ha-ras (T24) oncogene. They were also analyzed for their ability to form antibiotic resistant colonies when transfected with a cloned neomycin resistance gene (pSV2neo) and selected for growth in medium containing G418. CREF-Trans 6 is a specific subclone of CREF which was found to be more sensitive than parental or other CREF-Trans subclones to transformation by T24 and for the development of neomycin resistance.

Other Cell Lines. The LN-CaP cell line was derived from a human prostatic carcinoma. The T47D cell line was derived from a human breast carcinoma. The GBM-18 cell line was derived from a human glioblastoma multiform (stage IV astrocytoma). There was also a human glioblastoma multiform (stage IV multiform) derived from a primary tumor. The SW480 cell line was derived from a human colorectal carcinoma. The MCF-7 cell line was derived from a human blast carcinoma. The Colo38 cell line was derived from a human melanoma. The HO-I cell line was derived from a human melanoma. These cell lines are publicly available to those of ordinary skill in the art.

Preparation of Anti-Serum. BALB/c female mice (8 to 10 weeks old) were hyperimmunized with CREF-Trans 6 cells. They received: (1) one subcutaneous (sc) injection of scraped cells with complete Freund's adjuvant (1:1) on day 0; (2) one sc injection of scraped cells with incomplete Freund's adjuvant (1:1) on day 7; and (3) two intraperitoneal (ip) injections of scraped cells in Hanks' balanced salt solution on days 14 and 21. Mice were then bled from the retro-orbital eye socket and the pooled sera was tested for anti-CREF activity by ELISA. CREF cells were: (1) grown in 96-well microtiter plates to near confluency; (2) the cells were fixed with 3.7% formaldehyde in PBS (5 min. at room temperature) and blocked with 10% normal goat serum; (3) antisera was titered against the cells (serial dilutions; 2 hrs. at 37°C); (4) binding was detected by using a goat anti-mouse Ig secondary antibody conjugated to horseradish peroxidase (G X MIg-HRP; 60 min; 37°C); and (5) a chromogen was added in the presence of H₂O₂ and a positive binding was reflected by a color change which was quantitated using a spectrophotometer. By employing the above procedures a high titer serum was obtained by day 21 (1:3200 - 1:6400) and used at a 1:100 for coating CREF-Trans 6 cells in initial experiments. A second high titer antiserum (1:12400) was used in subsequent experiments.

Cotransfection of the CREF-Trans 6 Cell Line. DNA transfections of recipient CREF cells have been performed as described previously (22,45,47), except the HMW-DNA has been sheared to produce DNA fragments of 6 to 25 kilobase pairs. This modification results in a more efficient transfer of certain cellular genes by calcium phosphate mediated DNA transfection and also tends to diminish the toxicity often encountered when transfecting HMW-DNA (11). DNA was then isolated from the cell lines using the phenol extraction method (14, 37). This HMW-DNA was then sheared by repeated passage through a syringe containing a 16-gauge needle and then sized by electrophoresis through agarose gels (36). The HMW-DNA was then mixed with pSV2-Neo DNA (using a 20 to 40 µg: 1 µg ratio of HMW-DNA: Neo plasmid DNA) and 20 or 40 µg of final DNA was added as a calcium phosphate precipitate to 1 to 2 x 10⁶ CREF-Trans cells (22,45,47). After incubation of CREF-Trans cells with the calcium phosphate-DNA precipitate for 4 hrs, the excess DNA-precipitate was removed, cultures were briefly treated with glycerol (15% glycerol: PBS vol/vol) and cultures were resuspended by brief trypsin/versene treatment and replated at 5 x 10⁴, 10⁵ and 2.5 x 10⁵ cells/10 cm plate.

Selection of transfected expressing the selectable or identifiable trait. Forty-eight hours post-plating, the medium will be replaced with medium containing G418 (500 µg/ml) (,36,45). The selective medium was changed two times per week and colonies of Neo^{R} should be detectable within 7 to 14 days.

Recovery and injection of transfected cells in mice. After cell cultures from individual plates have been expanded to adequate numbers, the cells from each separate G418 selected plate were pooled and 10⁶ were injected subcutaneously per nude mouse.

Isolating and obtaining the tumor cells. If the injection of the transfected cells induces tumors in mice during an eight week incubation period, the tumors will be removed and reestablished in cell culture. The cells were removed from plates by either non-enzymatic dissociation or by scraping with a rubber policeman. Resuspended cells were washed with Hanks BSS and resuspended in a minimal volume of Hank's BSS.

Coating the tumor cells with antiserum. In order to block antigens normally expressed on CREF-Trans 6 cells from generating an immune response, tumor cells from the tumor were coated with high titer mouse anti-CREF-Trans 6 antisera prior to injection into mice. Resuspended cells (1 to 2.5 x 10⁶ cells) were mixed with M X CREF anti-sera at a concentration of 1:100 (antisera:cells) and incubated for 4 to 6 hrs at room temperature or overnight at 4°C on a shaker.

Injecting the antiserum-coated cells into mice. BALB/c female mice (8 to 10 weeks old) were then repeatedly injected ip with and without adjuvant over a month period, or longer with antisera-coated cells.

Screening of hosts for serum reactive with neoplastic cell. Pooled bleeds of mice immunized as described above were tested by ELISA for binding to various cells, including CREF-Trans 6, CREF 4-NMT (transfected with LNCaP DNA), LNCaP (human prostatic carcinoma cell line), SW480 (human colorectal carcinoma cell line), MCF-7 (human breast carcinoma cell line), H0-1 (human melanoma cell line), Colo 38 (human melanoma cell line), and human skin fibroblasts. Animals which were identified as having sera reactive with the appropriate human tumor cells were then used to generate MoAbs by standard procedures.

Generation of Monoclonal Antibodies. The procedures utilized to generate MoAbs are similar to those described previously for generating MoAbs toward human TAAs (50), HLA antigens (51), type 5 adenovirus-transformed Sprague-Dawley rat embryo cells (52), X-ray-transformed C3H-10T 1/2 cells (53) and NIH-3T3 transfectants containing and expressing the neuro/glioblastoma (neu) oncogene (39). Spleen cells prepared from immunized mice were hybridized with the non-secreting myeloma cells NSI in the presence of polyethyleneglycol (PEG) according to the procedure of Kohler and Milstein (54) with minor modifications. Twenty-four hours after fusion the hybrid clones were replated in 96 well plates in HAT medium to select for hybrid cells. The supernatant of microtiter wells containing expanded hybrid clones were then collected and tested for antibody activity against multiple target cells, including tertiary and secondary LN-CAP-CREF transfectants and normal CREF cells. For initial screening, a modification of the Terasaki plate ¹²⁵I-protein A binding assay (55) was used because it requires a minimal number of target cells and the procedure is not time consuming. Other assay methods, e.g enzyme-linked immunoassay, are also available. Following initial screening, the hybridoma culture supernatant which reacted specifically with the LN-CaP-CREF transfectants was retested for specificity against a larger panel of target cells which may include, the prostatic carcinoma cell lines DU-145, PC-3, two melanoma cell lines (Colo 38 and HO-1 cells), a B lymphoblastoid cell line (WIL-2), normal human skin fibroblasts, and CREF-Trans 6 cells transformed by other agents (type 5 adenovirus, bovine papilloma virus type 1 and Ha-ras). The hybridomas that secrete antibody specific for the prostate tumor cell lines may be immediately subcloned at least 5 times by limiting dilution. The final hybridoma clones of interest, i.e. those which produce antibody which reacts specifically with human prostate cainoma TAAs, may be expanded in vitro into mass cultures or in vivo as ascites in pristane primed syngeneic mice. Antibodies of interest will then be used to characterize biochemically the prostate tumor-inducing antigens expressed on transfected CREF cells and human prostatic carcinoma cells.

Identification Of Specific Antigens using Antibodies. MoAbs of interest will be used to isolate specific antigen(s) from human prostatic carcinoma cells for biochemical analysis using procedures described previously. Initially, simple experiments may be carried out to determine the nature of the molecules (i.e. protein or glycolipid) bearing the epitopes recognized by the MoAbs. To identify protein or glycoprotein antigens, a radioimmuniprecipitation assay (56,57) may be used: Detergent extracts of cell membranes from cells labeled with ¹²⁵I or cells synthetically labeled with ³⁵S-methionine or ³H-leucine will be reacted with MoAb bound to anti-mouse (IgG + IgM)-sepharose; and the MoAb-antigen interaction will be analyzed by SDS-PAGE (56,57). To identify glycolipid antigens, the lipid components isolated from human prostatic carcinoma cells may be resolved by thin-layer chromatography and the chromatogram will then be reacted with the relevant MoAb.

The molecular weight and charge heterogeneity of the protein antigens will be further analyzed by two dimensional (2-D) gel electrophoresis as previously described by O'Farrell (58) and modified by Garrels (59). The antigens isolated by immunoprecipitation with MoAbs may then be subjected to isoelectric focusing in the first dimension in polyacrylamide gels containing ampholines and urea. The gels may then be equilibrated with SDS-gel buffer and applied to an SDS-PAGE gel. The charge heterogeneity due to sialic acid will be assessed by removing these residues with neuraminidase prior to 2-D analysis. The 2-D analysis thus provides simultaneously the isoelectric point (pI) and the molecular weight of proteins being analyzed.

To determine the stability of the protein antigen recognized by the MoAb, Western blotting analysis may performed. Briefly, cellular extracts denatured by SDS detergent may be resolved by SDS-PAGE under reducing or non-reducing conditions, blotted onto nitrocellulose membranes and stained with selected MoAbs using the immunoperoxidase method (blot will be treated with MAb followed by a peroxidase conjugated anti-mouse Ig antibody followed by H₂O₂ plus 3, 3'-diaminobenzidine tetrahydrochloride) or the blotted antigen will be bound with purified MoAb followed by ¹²⁵I-labeled Staphylococcus aureus protein A and autoradiography. These procedures will indicate: (a) whether the epitope remains immunoreactive following the treatment protocol; and (b) whether the protein antigen consists of subunits. Moreover, these studies will indicate whether the immunoblotting assay can be applied in the detection of the specific antigen(s) in patients' specimens.

Recovering DNA from the tumor cells. Using the tumor cells derived from the tumors, DNA can be recovered, identified, isolated and molecularly cloned employing known procedures. Southern blot analyses will determine if human DNA sequences are present within DNA extracted from tumor-derived cell lines that are progeny of primary, secondary, or tertiary transfections of the CREF-Trans 6 cells. Usually there are high numbers of human DNA-containing fragments (Alu) in restriction endonuclease digested DNA from primary rat or mouse transfected cells, while the secondary and tertiary transfectants yield few Alu DNA-fragments, one or no bands on a Southern blot probed using human Alu DNA. In Southern blot analyses, the 265 bp Alu DNA sequence, Blur-8 (23), that has been subcloned into a vector containing an SP6 promoter (provided by V. Racaniello) was used in order to generate ³²P-labeled probe RNA of high specific activity. Due to the small size of the Blur 8 probe, it is possible not to detect single copy representations of the Alu repeat integrated within heterologous species DNA. To determine if Alu DNA sequences can be detected in transfected rat cells containing only a single copy of Alu DNA, DNA from a cell line provided by V. Racaniello, Columbia University, which has previously been shown to contain a single copy of Alu DNA, has been included as a hybridization control. If Southern blot analyses reveal the presence of conserved human Alu DNA containing restriction fragments in multiple secondary or tertiary transfectants, it should be possible to isolate the human prostatic carcinoma genes responsible for tumorigenic conversion of CREF cells. This will be accomplished by the generation of genomic DNA libraries, isolation of cloned DNA-containing Alu DNA sequences, and subsequently testing the biological activity of these cloned human DNAs by cotransfecting CREF cells with pSV2-Neo DNA and determining if G418-resistant transfectants are now tumorigenic in nude mice.

Partial MboI digests of DNA extracted from tumor-derived secondary or tertiary LN-CAP DNA transfected CREF cells may be used in the construction of lambda EMPL3 (BamHI site) libraries (26) and these libraries may be screened by plaque hybridization (27) using the Blur-8 Alu DNA probe. DNA may then be extracted from recombinant lambda phage that contain Alu sequences and cloned human DNA will be assayed for its tumor inducing ability by cotransfecting CREF cells with pSV2-Neo DNA and scoring the G418-resistant population for tumorigenic potential in nude mice after subcutaneous injection of 10⁶ cells. If a recombinant phage contains a tumor-inducing gene from LN-CAP prostatic carcinoma cells, the cloned DNA may be mapped using restriction endonucleases, and the smallest fragment of cloned DNA which retains the ability to transform CREF cells to an oncogenic phenotype will be used to determine, in LN-CAP and normal human prostate tissues: (a) the newly isolated gene transcription rate by nuclear run-off assay of the newly isolated gene; (b) the level and abundance of introns homologous poly A⁺ RNA by Northern blot analysis; (c) the intron/exon arrangement using S₁ analysis (28); and, (d) using Southern blot analysis, if the DNA arrangement of the prostatic oncogene in LN-CaP cells is identical to the DNA arrangement in the genome of normal human prostate cells. Comparison, by Southern analysis, of normal, LN-CaP and primary, secondary, and tertiary LN-CaP transfected CREF cells will also permit one to determine if there is amplification of the genes responsible for the oncogenic phenotype of these prostatic carcinoma cells.

If there are recombinant phage from the secondary or tertiary transfectants that contain Alu DNA, but do not transform CREF-6 cells to the tumorigenic state in nude mice, these recombinant phage will be used to screen cosmid (pJB8) (27) and lambda EMBL3 phage libraries constructed from LN-CAP DNA partially digested with MboI or EcoRI. DNA contained in phage or cosmids that hybridizes to the probe generated from the original transfectant DNA/lambda libraries may then be mapped extensively using restriction endonucleases and DNA within the phage or the cosmids that appears to contain homologous sequences, in addition to flanking DNA, may then be used to transfect CREF-Trans 6 cells followed by analysis of transfectants for tumorigenic potential in nude mice. Those cellular DNA sequences within phage or cosmids that generate transfectants scoring positive in the nude mouse tumorigenicity assay may then be digested with various restriction endonucleases to determine the smallest DNA sequence responsible for tumor-inducing activity.

If there are no human Alu DNA-containing sequences cloned within a single phage or cosmid constructed from the secondary or tertiary transfectants that can convert CREF cells to an oncogenic phenotype, one may attempt to complement the oncogene by supplying an overlapping fragment from a normal human or LN-CAP DNA lambda EMBL3 or cosmid library generated by partial digestion using either sail or EcoRI instead of MboI, thus, allowing in vivo recombination to reconstruct the functional oncogene. Recently, this technique has been used to generate biological activity, for the human nerve growth factor receptor, by transfecting DNA defining part of this gene with DNA sequences that were selected from a normal human lambda library that hybridize to the partial gene encoding this receptor (24). The resulting transfectants expressed a new growth factor receptor that exhibited NGF binding activity (24). This approach may be useful if the above approaches are negative in the isolation of gene(s) responsible for oncogenic transformation of CREF cells.

It is possible that the gene(s) responsible for oncogenic conversion of CREF-Trans 6 cells can not be isolated using genomic clones and searching for this human gene by virtue of its close association with human Alu sequences. As an alternative to the initial approach, one may base the gene isolation strategies upon isolation of cDNAs reflecting different mRNA species or mRNA levels among highly related non-oncogenic CREF cells and the tertiary transfectants of these CREF cells displaying an oncogenic phenotype. The difference between CREF cell gene expression and tumorigenic LN-CAP transfectants is likely due to the expression of genes from human prostatic carcinoma DNA. Based on this hypothesis, RNA from cloned tertiary transfectants may be used to construct cDNA/lambda-gt10 libraries and these libraries will be screened with ³²P-labeled cDNA probes generated from CREF mRNA or CREF transfectant mRNA.

Ten micrograms of poly A⁺ RNA will be sufficient to construct libraries which should contain 10⁶ independent recombinant phage. λgt10-cDNA libraries of this size should contain sequences present at levels of less than 0.001% of the total mRNA. λgt10-cDNA libraries constructed from tertiary LN-CaP CREF cell transfectants will be screened by a number of strategies (comparative, competitive and subtractive screening (11-13,29,64) to isolate cDNA clones representing mRNAs expressed uniquely or at height levels in CREF-transfectants compared to CREF cells.

To screen the libraries for genes expressed in LN-CaP-transfected CREF cells but not in CREF-Trans 6 cells, duplicate nitrocellulose filters containing the appropriate recombinant phage-tertiary CREF transfectant cDNA may be hybridized to ³²P-labeled cDNA probes made from untransformed CREF-Trans 6 cells and tumor-inducing LN-CaP transfected CREF cells (64). As a second screening approach, nitrocellulose filters containing recombinant phage DNA molecules (containing cloned cDNAs from CREF-Trans 6 or CREF-Trans 6 cells transfected using LN-CaP DNA) may be hybridized to ³²P-labeled cDNA probes prepared from tertiary transfectant CREF cells in the presence of several hundredfold excess of other CREF mRNA (11). As a third approach one may also use subtractive screening procedures in which nitrocellulose filters containing recombinant phage-tertiary transfectant DNA molecules are hybridized to ³²P-labeled cDNA probes enriched in untransformed CREF cell sequences or tertiary CREF cell cDNAs (12,13). Using these procedures it is possible to isolate specific recombinant phage which contain gene sequences that are uniquely expressed or over expressed in transfected CREF cells compared to CREF cells.

cDNA clones representing mRNA differentially expressed among tumorigenic and nontumorigenic cells may be assigned to homology groups by cross hybridization analysis and mapped using restriction endonucleases (29). The largest cDNA insert from each homology grouping will be used to characterize the size and abundance of homologous mRNAs, if present, from CREF-Trans 6 cells, LN-CAP cells and CREF-Trans 6 cells transfected using LN-CaP DNA to insure that the cloned cDNA represents human-specific mRNA and that it is from a gene that may be responsible for the oncogenic conversion of CREF-Trans 6 cells and the control of prostatic carcinoma. Northern blot analyses may also indicate if these genes are also expressed in: (a) normal prostate tissue, possibly in lower levels; (b) clinical tissue samples derived from prostate tumors manifesting different stages of progression of prostatic carcinoma in humans; and (c) prostatic cells from normal or cancerous tissue derived from rats.

Recombinant phage that contain cDNAs representing differentially expressed mRNAs may then be used as a probe in Northern blot analysis of mRNA extracted from CREF-Trans 6 cells in addition to mRNA from the cloned tertiary transfectants. If the Northern analyses identify genes expressed solely in the transfected cells, these cDNA clones may then be employed as a probe to identify cosmids of phage from genomic libraries of the tertiary transfectant DNA in order to isolate and identify gene sequences that control expression of these genes. Once the genomic counterparts are isolated, the structure of the oncogene(s) can be studied by the techniques of S₁ nuclease mapping (29), restriction endonucleases mapping (29), and by DNA sequencing (14).

To confirm the biological function of the cloned cDNA(s), the cDNA(s) may be excised from recombinant λgt10 DNA and the cDNA may be inserted into the pCD expression vector (15,16). The pCD-cDNA recombinant plasmids may then be cloned into a λ-NMT bacteriophage which contains the neomycin gene fused to an SV40 early gene transcription unit, thus conferring G418 resistance to mammalian cell transfectants and a strong promotor for cDNA expression (15). λNMT-pCD-cDNA recombinant bacteriophage particles may be used directly for high efficiency transfection of CREF cells. The G418-resistant population may be tested for tumorigenicity in nude mice and transformation-associated traits such as changes in cell morphology, growth and the ability to grow in agar-containing medium.

Identification of a gene(s) regulating the tumor-inducing phenotype in CREF cells from human prostatic carcinoma cells will be valuable in: (a) determining if a similar gene(s) is present and expressed in other human prostatic carcinoma cell lines, including DU-145 and PC-3; (b) determining if a similar gene(s) is present and/or expressed in normal human prostate, benign prostatic hypertrophy and prostatic carcinoma tissue representing different stages in the evolution of this disease; (c) determining if a homologous gene(s) is expressed in rat prostatic carcinoma cells and if expression of this gene is altered in cells exhibiting different degrees of metastatic potential; (d) identifying and characterizing specific protein(s) which may be involved in mediating the tumor-inducing phenotype in CREF cells, and (e) generating defined immunological reagents which might be useful in the diagnosis of specific stages of prostate cancer and in monitoring the course of therapy in patients receiving treatment for prostatic cancer.

### Identification and Cloning of the Tumor-Inducing Gene Transferred from a Human Prostatic Carcinoma Cell Line (LNCaP) to CREF Cells.

Attempts to demonstrate consistent changes in oncogene expression in primary and established human prostatic carcinoma cells have been unsuccessful (8-10). However, defects in expression of the retinoblastoma (RB) tumor suppressor gene (located on chromosome 13q14) have been found in the DU145 human prostatic carcinoma cell line (64) and 2 of 10 human prostatic carcinoma tumor specimens (65). Specific allelic loses in chromosomes 16q and 10q may also be involved in the pathogenesis of human prostate cancer (66-68). The role of genetic alterations in RB and other putative tumor suppressor genes (located on chromosomes 16q and 10q) in mediating the behavior of a specific prostatic carcinoma or in determining patient prognosis is not known (64-68). In addition, further studies are required to determine if alterations in tumor suppressor gene(s) by themselves induce prostatic carcinomas or if these genetic alterations occur concomitantly with the activation of yet to be identified oncogenes. Calcium mediated DNA-transfection of high molecular weight DNA from prostatic carcinoma cells into NIH 3T3 cells has failed to identify a common dominant-acting focus-inducing oncogene in this class of tumors (9). A refinement of the single DNA-transfection technique has permitted the identification of additional oncogenes, including those with weak transforming potential, in histologically different human tumors (12,13,68-71). This procedure involves cotransfection of established cell lines with human tumor DNA and a selectable antibiotic resistance gene, selection for antibiotic resistance and injection of resistant colonies into nude mice (12,13,69-71). Cotransfection of CREF or NIH 3T3 cells with DNA from a cloned neomycin resistance gene (pSV2neo) plus high molecular weight DNA from LNCaP cells followed by selection for resistance to G418 did not result in morphologically transformed foci. However, injection of cotransfected CREF, but not cotransfected NIH 3T3 cells, into nude mice resulted in tumor induction in 3 of 4 sets of animals receiving pooled cells from different transfections. Cotransfection of CREF cells with pSV2neo and DNA from normal skin fibroblasts or salmon sperm DNA did not result in tumor formation. DNA from primary LNCaP-CREF tumors (1° transfectants) induced the tumorigenic phenotype following a second round of cotransfection into CREF cells. Antibiotic resistant colonies selected following the second round of cotransfections (2° transfectants) did not display morphological transformation in vitro. However, these antibiotic resistant morphologically normal cultures induced tumors in 6 of 7 sets of animals receiving pooled cells from different transfections. Both 1° and 2° transfectants obtained from LNCaP-CREF- induced tumors in nude mice contained repetitive human (Alu) sequences which were not detected in CREF cells (several of these transfectants are shown in Figure 1). Both 1° (CREF 4 NMT) and 2° (CREF 4-5 NMT; CREF 4-7 NMT) transfectants contained apparently unique Alu fragments in addition to a common Alu fragment of approximately 7.5 kb. This observation suggests that the putative tumor-inducing gene derived from these human prostate carcinoma cells is located adjacent to this DNA sequence. As will be described below, these 1° and 2° transfectants can now be used to isolate the gene(s) mediating the tumor cell phenotype which have been transferred from the human prostatic carcinoma cell line LNCaP to CREF cells.

### Gene Cloning Strategies to be Employed to Isolate the Transfected Tumor-Inducing Gene from LNCaP Cells.

Cloning in EMBL3 Phage. The basic procedure employed is well established for identifying Alu Human repeat sequences and cloning these sequences which are present in human DNA-transfected rodent cells (23,70-72). In addition, this approach has recently been used to identify a potentially new oncogene from patients with familial adenomatous polyposis after cotransfection of NIH 3T3 cells with pSV2neo DNA and human cellular DNA followed by tumor formation in nude mice (71). As indicated in Figure 1, Southern blotting studies demonstrate that both 1° and 2° tumor-derived LNCaP DNA-CREF transfectants contain approximately a 7.5 kb hybridizing DNA fragment which is generated after cleavage with EcoR1 and probing with a Blur 8 (Alu) probe (23). To isolate a genomic clone which contains this putative LNCaP-tumor inducing gene, the following strategy may be employed. Seven to 9 kb DNA fragments of EcoRI digested DNA, which were positive when probed with Blur 8, will be isolated and extracted from low melting agarose gels by treatment with phenol, phenol: chloroform and chloroform. The DNA fragments will be ligated into bacteriophage EMBL3 arms cut with EcoRI (Stratagene, CA) (72). Following ligation, the reaction mixture will be packaged using Stratagene packaging extracts. The total phage library will be plated onto LE392 host bacteria and Alu positive plaques will be identified by plaque hybridization (73) on nitrocellulose paper with Blur 8 as a probe (74,75). The Alu probe will be labeled using the random oligonucleotide labelling procedure (76) with [α-³²P] dCTP (Amersham, Inc., IL). Positive genomic clones will be characterized by restriction enzyme mapping and Southern blotting analysis (77,78). Positive clones will be subcloned into pUC19 or M13 phage for sequencing and they will also be used as probes for Northern blotting studies. For Northern blotting studies, cytoplasmic RNA isolated from LNCaP, 1° and 2° tumor-derived LNCaP DNA-CREF transfectants and additional human prostatic carcinoma cell lines (including DU145 and PC-3) will be used. Clones which are positive in these Northern blotting assays and which contain exons of similar size will be further characterized by sequencing. Appropriate exon containing clones will be used to screen cDNA libraries constructed from LNCaP and 1° and 2° tumor-derived LNCaP DNA-CREF transfectants to obtain a full length cDNA clone corresponding to the putative tumor-inducing gene.

As a second approach employing EMBL3 cloning to obtain the putative tumor-inducing gene present in 1° and 2° tumor-derived LNCaP DNA-CREF transfectants the following approach will be employed (71,74,76). Secondary tumor-derived LNCaP DNA-CREF transfectant DNA will be partially digested with Sau3AI and then fractionated by sucrose gradient centrifugation. The purified fraction containing the 20 to 30 kb fragments will be ligated with purified EMBL3 phage arms cut with BamHI. The recombinant phage library will be screened with an Alu probe and positive clones will be identified. The cloned DNAs will then be digested with SalI and analyzed by Southern blotting with an Alu probe and the probe obtained above.

Polymerase Chain Reaction (PCR) Cloning. Isolation of human DNA sequences from 1° and 2° tumor-derived LNCaP DNA-CREF transfectants using recombinant phages will require the production of a total genomic library (containing 2 to 4 X 10⁵ independent recombinant phages) in EMBL3 and screening the library with Alu as a probe. Recent studies indicate that the Alu PCR approach can be used as a simple method to identify and purify human DNA sequences from complex sources such as somatic cell hybrids retaining human chromosome fragments in a rodent genomic background, genomic DNAs cloned in lambda phage and yeast artificial chromosome (YAC) vectors (79,80). This approach has been facilitated by using a consensus sequence of the Alu repeat which is reasonably well conserved in the human genome (79). The PCR primer consensus sequences which have proven of particular value in identifying human DNA sequences in somatic cell hybrids and YAC vectors are TC-65 and 517 (79). Primer TC-65 is located within a 31 bp sequence that is unique to the second monomer of primate Alu repeats (81). Primer 517 recognizes the same 17 bp of Alu sequence as TC-65 but in the opposite direction. Based on the studies of Nelson et al. (79) it was estimated that primer TC-65 will identify a fragment produced for each 500kb of human DNA and primer 517 will produce a fragment every 1000 kb. If these consensus Alu sequences are present in 1° and 2° tumor-derived LNCaP DNA-CREF transfectants then the Alu PCR approach will eliminate the requirement of preparing a genomic library and screening this library for Alu positive clones. Initially, 1µg of 1° and 2° tumor-derived LNCaP DNA-CREF transfectant DNA and PCR using the TC-65 and 517 primers as described by Nelson et al (79) will be employed. Modifications of this approach, either through the use of primers permitting bidirectional amplification from a single Alu repeat using two primers or by inverted PCR (82), should allow amplification of the majority of Alu sequences from a region or clone. If the PCR Alu methodology is successful, the sequences that are identified can be used directly as probes for use in screening recombinant libraries derived from 1° and 2° tumor-derived LNCaP DNA-CREF transfectants to identify cDNA clones of interest.

Differential Screening (Subtractive Screening). Screening subtracted libraries constructed from 2° tumor-derived LNCaP DNA-CREF transfectants will result in a greater enrichment for hybridization positive phage clones than can be obtained from screening a total 1 X 10⁶ recombinant phage cDNA library. A unidirectional cDNA expression library from CREF and 2° tumor-derived LNCaP DNA-CREF transfectant cells will first be prepared from 5 µg of Poly A+ RNA and then a double stranded cDNA will be prepared with the Stratagene cDNA synthesis kit (83). The cDNAs will be ligated into Lambdazap bacteriophage vector and packaged with packaging extracts. A total of 1 X 10⁶ independent clones will be scored. Subtracted libraries will be made from the above cDNA libraries by the method of Sargent and Dawid (84). Thirty µg of 2° tumor-derived LNCaP DNA-CREF transfectant cDNA present in Lambdazap vector will be cleaved with XhoI and RNA transcripts will be generated by T3 RNA polymerase (85). The RNA transcripts will be converted into single strand cDNA by Moloney murine leukemia virus reverse transcriptase with the addition of XhoI linker primers. This single strand cDNA will be hybridized with excess of CREF cytoplasmic RNA at 60°C for 48 hr. The unhybridized cDNA will be recovered by passing through a hydroxyapatite column (86). Approximately two rounds of subtraction will result in 90% subtraction from the 2° tumor-derived LNCaP DNA-CREF transfectant cDNA. This subtracted cDNA can be used as a probe or it can be further processed to double stranded cDNA with Klenow polymerase, ligated to phage vectors and packaged. The total number of colonies obtained should be 5-10 X 10⁴. This approach is optional and would only be employed if for some reason attempts to identify the putative LNCaP tumor-inducing gene are unsuccessful using EMBL3 cloning or the PCR Alu procedure.

An additional approach which could theoretically be used to clone the LNCaP tumor-inducing gene from CREF transfectants combines both subtractive hybridization and PCR techniques (87). In this procedure, a large excess of driver DNA (CREF) is combined with a tester DNA (2° tumor-derived LNCaP DNA-CREF transfectant which contains the gene(s) of interest which is not present in the driver DNA) and by subtractive procedures common sequences are removed resulting in an enrichment in the target DNA (in the-tester DNA sample) sequence. After separation of subtracted tester DNA from driver DNA by avidin/biotin affinity chromatography the single-stranded target DNA is amplified by PCR rendering it double-stranded and clonable. Using this new methodology, Wieland et al. (87) have produced a 100- to 700-fold enrichment of target DNA sequences. Employing this approach it should be possible to subtract away common sequences present in both CREF and 2° tumor-derived LNCaP DNA-CREF transfectants resulting in an enrichment in Alu linked sequences present only in 2° tumor-derived LNCaP DNA-CREF transfectants. These Alu linked sequences can then be amplified using PCR and the gene(s) can be directly cloned into pUC18 vectors for further analysis.

Lambdazap Expression Vector and Probing with Polyclonal Antibodies. Polyclonal and monoclonal antibodies reactive with LNCaP cells by using a high titered CREF antisera to coat 1° tumor-derived LNCaP DNA-CREF transfectants prior to immunization of mice have been developed. Using this approach and immunizing rabbits, polyclonal antibodies have been generated which are reactive with antigens expressed on the surface of 1° and 2° tumor-derived LNCaP DNA-CREF transfectant and LNCaP cells but not CREF cells. cDNA libraries from 1° and 2° tumor-derived LNCaP DNA-CREF transfectants and LNCaP cells would be constructed in Lambdazap expression vectors and colonies will be screened with polyclonal antibodies (neutralized against E. coli extracts (77) as described by Young and Davis (88). Approximately 1 X 10⁶ recombinant phage clones will be screened 3 to 4 successive times with polyclonal antibodies to obtain a positive clone. This cDNA will then be subcloned and sequenced as previously described (77,78). This approach is presented only as a further alternative approach for identifying and cloning the LNCaP tumor-inducing gene which has been transferred to CREF cells.

It is highly probable that the procedures outlined above will result in the isolation and identification of the putative prostatic carcinoma tumor-inducing or tumor-associated gene present in LNCaP cells and transferred via calcium-mediated DNA transfection into CREF cells.

### Organization and Expression of the Transfected Tumor-Inducing Gene from LNCaP Cells in Normal Prostate, Benign Prostatic Hypertrophy (BPH), Prostatic Carcinoma and Metastatic Tumors to Bone.

The procedures described above will result in the isolation of a cDNA or genomic clone which has been transferred from LNCaP cells to CREF cells and which mediates the tumor phenotype of transfected cells, referred to as prostate carcinoma tumor inducing/associated (PCTI) gene. Once identified, this clone will be used to determine its potential role in human prostatic carcinoma development. These studies will include: (a) an analysis of its expression in normal human prostate, BPH and prostatic carcinoma (of different Gleason stages) cell lines; (b) an analysis of its expression in primary tissue from normal human prostate, BPH, prostatic carcinoma (of different Gleason stages) and metastatic tumors to bone; and (c) determining if a homologous gene(s) is expressed in rat prostatic carcinoma cells and if expression of this gene is altered in cells exhibiting different degrees of tumorigenic and/or metastic potential.

Expression of the PCTI Gene in Human Prostate Cell Lines Representing Different Stages in Prostatic Carcinoma Development. The organization and expression of the PCTI gene derived from LNCaP DNA-transfected tumor-derived CREF cells in normal prostate, BPH and prostatic carcinoma cell lines (8) will be determined as previously described by Southern (6,71,74,75) and Northern blotting analysis (47,71). PCTI gene organization and expression will also be determined in additional normal cell lines and tumor-derived cell lines. These will include: normal human skin fibroblasts, epidermal keratinocytes, melanocytes and mammary and colon epithelial cells; and tumor-derived cell lines from sarcomas, hematologic malignancies, melanomas, central nervous system tumors and various carcinomas (breast, colorectal, etc.). Since the rat prostate model system, including the Dunning rat dorsal prostatic adenocarcinoma system (7,89), will prove useful in defining the functional properties of the PCTI gene these systems will also be evaluated. The above studies will provide information on the distribution of expression of the PCTI gene isolated from LNCaP DNA-transfected tumor-derived CREF cells.

Expression of the PCTI Gene in Primary Prostatic Tissue Representing Different Stages in Prostatic Carcinoma Development. It will be important to determine if the PCTI gene is expressed in vivo in human prostatic carcinoma cells. RNA will be extracted from normal prostate, BPH, various Gleason stage prostatic carcinomas and prostatic carcinomas which have metastasized to bone using previously described techniques (90). These RNAs will be analyzed by slot-blot (90) and Northern blotting (47) to determine levels of expression of the PCTI gene. To normalize for RNA expression and loading, blots will be reprobed with glyceraldehyde phosphate dehydrogenase (GAPDH).

If the PCTI gene is differentially expressed in primary prostatic tissue, this gene will be subcloned into a pGEM-4 or SK vector and determine by in situ hybridization (91) the expression of the PCTI gene in frozen tissue sections from normal prostate, BPH and prostatic carcinoma tissue. By employing rodent-human hybrids which have retained specific human chromosomes and Southern blotting analysis, studies will also be conducted to determine the chromosomal location of the PCTI gene in the human genome (71).

### Functional Studies of the PCTI Gene Derived from Human Prostatic Carcinoma (LNCaP) Cells.

A critical issue will be to determine if the PCTI gene isolated can function as a tumor-inducing gene which expressed in CREF cells and/or if this gene can function as a tumor-inducing gene when expressed in normal human, rat and mouse prostate cells. Studies will also be conducted to determine if the PCTI gene can alter the phenotype, as indicated by growth in agar and tumorigenicity in nude mice, of human BPH and human, rat and mouse prostatic carcinoma cells. A useful model system to determine if the PCTI gene can cooperate with other oncogenes, such as ras and myc, in inducing prostatic carcinomas is the mouse reconstituted organ system described by Thompson et al. (92). In this system, ras expression in normal mouse prostate cells results in dysplasia in combination with angiogenesis, myc induces a hyperplasia of the otherwise normally developed prostate and ras plus myc together induce primarily carcinomas (92).

DNA Transfection and Expression Studies. Initially, the PCTI gene will be inserted into an appropriate mammalian expression vector system containing a neomycin resistance gene (and a replication defective retroviral vector containing a neomycin resistance gene), transfected (or infected in the case of the retrovirus) into CREF cells and neomycin resistant colonies will be selected and injected into nude mice. If these transfected cells expressing the PCTI gene induce tumors, the tumors will be excised and analyzed for the presence of the appropriate gene (Southern analysis) (14) and its expression (Northern analysis) (47). It can also be determined whether tumor-derived CREF cells expressing the PCTI gene express TAAs on their cell surface which are recognized by the MoAbs developed above. A positive result would support the hypothesis that the cloned gene is the tumor-inducing genetic element which had been officially transferred to CREF cells from LNCaP cells.

Secondly, whether the expression of the PCTI gene in normal mouse, rat and human prostrate cells can induce expression of a partial (immortality) or a complete (anchorage independence and tumorigenic potential in nude mice) transformed phenotype will be determined. These studies will be conducted by transfecting the PCTI gene into appropriate target cells, selecting for G418 resistance and isolating individual clones (93). These clones will then be analyzed biologically (1) and molecularly (14,47,93) to determine if they contain and express the PCTI gene and if they have acquired alterations in phenotype. The tumorigenic and metastatic potential of these transfected prostate cells in nude mice will also be determined using previously described protocols (1,95). Similar studies will also be conducted in human BPH cells to determine if the PCTI gene can progress these cells to a tumorigenic and/or metastatic state. By expressing the PCTI gene in Dunning rat prostate adenocarcinoma and human prostate carcinoma cells it will be possible to further explore the role of the PCTI gene in regulating tumorigenic and metastatic potential of transfected cells in nude mice (1,95). Experiments will also be conducted as described by Thompson et al. (92) to determine if the PTCI gene can by itself, or in combination with either ras or myc, induce carcinomas in reconstituted mouse prostate glands. These studies described above will provide valuable information on the putative functional role of the PTCI gene in the development of human prostate carcinoma.

### Characterization of Monoclonal Antibodies (MoAbs) Developed Against LNCaP DNA-Transfected Tumor-Derived CREF Cells.

The neoplastic phenotype is often characterized by the surface expression of novel tumor associated antigen (TAA) subsets which are specific for different histological types of tumors (38). It is possible that in certain cases the genetic element(s) which induce the tumorigenic phenotype may also encode these specific cell surface TAAs. A direct relationship between the PCTI gene transferred into CREF cells and expression of the prostatic carcinoma phenotype is suggested by the ability to use LNCaP DNA transfected tumor-derived CREF cells to generate MoAbs which react with LNCaP cells. LNCaP DNA-transfected tumor-derived CREF cells were coated with high specific activity CREF polyclonal antisera and injected into mice. Sera from these animals contained polyclonal antibodies which reacted with LNCaP cells and the spleens from these animals were used to generate MoAbs which specifically interact with human prostatic carcinoma cells, including LNCaP and PC3. These same MoAbs do not cross-react with CREF or CREF cells transfected with DNA from other histologically distinct human tumors nor do they interact with normal human skin fibroblasts, melanoma, breast carcinoma, glioblastoma multiforme or colo-rectal carcinoma. MoAbs have also been isolated using the above approach which interact with LNCaP, but which do not cross-react with the cell types listed above or with PC3 cells.

In previous studies, NIH 3T3 cells transfected with DNA from an acute lymphocytic leukemia (ALL) (96) or a pancreatic adenocarcinoma cell line (HPAF) (97) produced morphologically transformed foci. These morphologically transformed NIH 3T3 cells were then used to develop MoAbs which did not react with NIH 3T3 cells, but which bound to epitopes on the cell membranes of histologically similar human tissue. In the case of the ALL-DNA transfected/transformed NIH 3T3 derived MoAbs, reactivity was observed with ALL-DNA transfected/transformed NIH 3T3 cells as well as fresh human leukemias, cultured leukemia cell lines. T-cell lines and normal hemopoietic cells (96). In the case of the HPAF-DNA transfected/transformed NIH 3T3 derived MoAbs, reactivity was found with the HPAF cell line as well as six additional human pancreatic adenocarcinoma cell lines. In contrast, the HPAF-DNA transfected/transformed NIH 3T3 derived MoAbs did not exhibit cross-reactivity with lymphoblastoid tumors (myeloid (K562 and HL-60), T-cell (CEM, MOLT and RESKW3), B-cell (SB, Daudi and WIL-2) and CALLA), melanomas, prostatic carcinoma or normal (skin fibroblast and normal pancreas) cell lines (97). These experiments and studies described above employing MoAbs generated against tumor derived LNCaP DNA-transfected CREF cells suggest that: (a) human tumor associated antigens can be transferred and expressed in heterologous mouse and rat cells; and (b) transfected cells can be used to generate MoAbs which display specific and restricted interactions with human tissue of different histotypes. Experiments described below are designed to further characterize the MoAbs generated against LNCaP DNA-transfected tumor derived CREF cells as potential tools for the diagnosis and ultimately the therapy of prostatic carcinoma.

The MoAbs developed which interact with LNCaP cells will be characterized as described previously (98). These studies will include an extensive analysis of reactivity of the MoAbs by ELISA with Formalin-fixed normal (prostatic epithelial, colonic epithelial, mammary epithelial, etc.) and tumor-derived human (BPH, prostatic carcinoma, metastic prostatic carcinoma from bone, colo-rectal carcinoma, breast carcinoma, etc.) cell lines. Additional studies will be conducted as previously described (98-100) to determine: (a) if the antigenic epitope reactive with the MoAbs is shed into the culture fluid; (b) the expression by FACS analysis of the MoAbs in viable and permeabilized normal prostate, BPH and prostatic carcinoma cells; (c) the molecular weights of the epitopes recognized by the MoAbs using PAGE and Western blot analysis; and (d) the biochemical nature of the antigens recognized by the MoAbs using various enzymes (such as fucosidase, β-glucosidase, β-galactosidase, mixed glycosidases, periodic acid, mannosidase, neuraminidase and protease). The MoAbs will also be compared with prostate-specific antigen (PA) and prostatic acid phosphatase (PAP) MoAbs with respect to identification of prostatic and additional cell types. PA and PAP are not useful as early markers of prostate cancer, but they are useful in monitoring disease recurrence and treatment response (101). It will be interesting, therefore, to determine how the MoAbs compare with these established immunological reagents and if the epitopes recognized by the MoAbs are present in normal prostate, BPH and/or prostatic carcinoma cells.

Reactivity Spectra of MoAbs Developed Against LNCaP DNA-Transfected Tumor-Derived CREF Cells Toward Human Tissue. It will be important to determine if the MoAbs developed against LNCaP DNA-transfected tumor-derived CREF cells can recognize antigenic epitopes on prostatic carcinoma tissue from patients. Studies will therefore be conducted to ascertain whether the MoAbs can identify prostatic cells in frozen and formalin-fixed/paraffin-embedded tissue from normal prostate, BPH, prostatic carcinoma and sections of bone containing prostatic carcinoma metastases. If the MoAbs can detect shed antigenic epitopes, it will be determined if urinary and semen secretions and blood samples from patients with prostatic carcinomas (of different Gleason grades) and patients with prostatic carcinomas which have metastasized to bone contain these antigens. Similarly, if a good correlation is observed in the expression of antigenic epitopes recognized by the MoAbs and prostatic carcinoma cells, it can also be determined if the MoAbs can or cannot interact with a wide spectrum of additional normal and tumor cells of different histological origins. For all of these studies, PA and PAP will be used as appropriate comparative control reagents. These studies will be necessary to determine if the MoAbs developed can be useful for the diagnosis of prostatic cancer in humans and if they offer any selective advantage over PA and PAP MoAbs.

Biological Studies to Address the Potential Function of the TAAs Recognized by MoAbs Developed Against LNCaP DNA-transfected Tumor-Derived CREF Cells. At present, the role of the PCTI gene transferred into CREF cells from LNCaP cells is not known. To begin to address this issue, the LNCaP and LNCaP DNA-transfected tumor-derived CREF cells will be coated with the MoAbs to determine if the growth in monolayer and/or agar suspension is altered (94). MoAb-coated cells will also be evaluated for changes in tumor development and growth after implantation into nude mice (97). As appropriate controls for the experiments described above, similar studies will be conducted with: (a) LNCaP and LNCaP DNA-transfected tumor-derived CREF cells coated with PA and PAP; and (b) LNCaP and LNCaP DNA-transfected tumor-derived CREF cells incubated with non-reactive antibody, such as the high molecular weight-melanoma associated antibody which does not bind to these cells (98,100). If any of the MoAbs suppress tumorigenesis of LNCaP cells in nude mice, the study will be expanded to include an evaluation of the effect of these MoAbs on additional prostatic carcinomas reactive with the MoAbs. Although negative results will not be informative, suppression of anchorage-independent growth and/or tumorigenesis by the MoAbs in prostatic carcinoma cells would provide strong evidence for a relationship between the PCTI gene-encoded TAAs and expression of the transformed state in human prostatic carcinoma cells. In addition, if a positive result is obtained the MoAbs identified could prove useful after conjugation with toxins or high energy radionuclides for therapy of prostatic cancer (102).

### Results and Discussion

CREF cells cotransfected with DNA from the human prostatic carcinoma cell line, LNCaP, and pSV2neo induce tumors in nude mice. Both 1° (first transfection) and 2° (second transfection) tumor-derived CREF cells contain human Alu sequences. All of the tumors contain apparently unique Alu fragments in addition to a common Alu fragment of approximately 7.5 kb (Figure 1). This observation suggests that the putative tumor-inducing gene derived from these human prostate carcinoma cells is located adjacent to this DNA sequence. Employing 1° and 2° tumors derived from nude mice injected with CREF cells transfected with human prostate DNA, various strategies can now be used to isolate the gene mediating the tumor cell phenotype. One approach would be to prepare genomic DNA libraries in EMBL3 phage using the DNA corresponding to Alu positive signals found in 1° and 2° tumor-derived CREF transfectants. Alu positive clones can then be identified, subcloned and the presence of exons can be determined by Northern blotting. Subclones hybridizing to similar size RNAs on Northern blots can be further characterized by sequencing and they can be used as a probe for screening either genomic or cDNA LNCaP libraries. A second approach would be to use Alu specific probes to identify and amplify DNA adjacent to these sequences in 1° and 2° tumor-derived CREF-Trans 6 transfectants by the polymerase chain reaction (PCR). These amplified DNA sequences can then be cloned directly into expression vector systems and tested for tumor-inducing potential in nude mice. A third approach would be to make subtracted libraries from CREF NMT 4 and CREF-Trans 6 cells. Additional approaches could be employed for identifying and cloning the gene in LNCaP cells which mediates tumor formation in CREF cells and which may be involved in prostatic carcinoma development in humans. These include: (1) Partially digesting LNCaP DNA with SauIIIAI, selecting DNAs of 30 to 35 kb and producing a genomic library in a genomic neo-expression vector system. These DNAs can then be transfected into CREF cells and neo-resistance and injecting pooled cell populations into nude mice for tumor formation. The likelihood of successfully identifying and cloning the putative tumor-inducing gene derived from LNCaP cells using the above strategies is high. In addition, the approaches outlined above can also be used to identify and clone other tumor-inducing genes from other specific human malignancies which have been transfected and expressed in CREF-Trans 6 cells. Once identified, the putative tumor-inducing gene from LNCaP (and other identified, the putative tumor-inducing gene from LNCaP (and other tumors) can then be used as a probe to determine its organization and expression in normal prostate, benign prostatic hypertrophy and different Gleason stage prostatic carcinomas in humans. The potential role of this tumor-inducing gene from LNCap cells in mediating metastasis in prostatic carcinoma cells could also be addressed.

As indicated above, the neoplastic phenotype is often characterized by the surface expression of novel tumor associated antigen (TAA) subsets which are specific for different histological types of tumors. It is possible that in certain cases, the genetic element(s) which induce the tumorigenic phenotype may also encode these specific cell surface TAAs. In a number of cases, TAAs have proven to be suitable targets for MoAb-based therapy resulting in direct tumor growth suppression. The possible relationship between induction of the tumor cell phenotype and expression of specific TAAs has been directly tested using LNCaP transfected-tumor-derived CREF-Trans 6 cells (CREF 4 NMT). By injecting mice with CREF 4 NMT cells coated with a high specific activity polyclonal antibody generated against CREF-Trans 6 cells, both polyclonal and monoclonal antibodies interacting with antigens expressed on CREF 4 NMT and LNCaP cells (Table 1 and Figs. 2, 3 and 4) have been successfully generated. CREF-Trans 6 cells generated an

**TABLE 1**

| | | **MOUSE ANTISERA** **GENERATED AGAINST** | |
|---|---|---|---|
| **CELL LINE** | **ORIGIN** | **CREF** | **CREF-NMT** |
| CREF-Trans 6 | Rat embryo Fibroblast | + | - |
| | | | |
| CREF-NMT 4 | LNCaP-Transfected Tumor-Derived CREF | + | + |
| | | | |
| LNCaP | Human Prostatic Carcinoma | - | + |
| | | | |
| SW480 | Human Colo-Rectal Carcinoma | - | + |
| | | | |
| MCF-7 | Human Breast Carcinoma | - | + |
| | | | |
| Colo38 | Human Melanoma | - | - |

immune response to CREF-Trans 6 and CREF 4 NMT cells, but not to cells of human origin. In contrast, CREF-Trans 6 coated CREF 4 NMT cells generated a polyclonal antibody response which identified antigens on CREF 4 NMT, LNCaP, human colo-rectal carcinoma (SW480) and human breast carcinoma (MCF-7), but not on CREF-Trans 6, human skin fibroblasts (WI-38) or human melanoma (HO-1) cells (Table 4 and Fig. 2). Further characterization, by titration analysis, of the polyclonal antibody produced by injecting mice with CREF-Trans 6 coated CREF 4 NMT cells is shown in Fig. 3. As can be seen, the anti-CREF 4 NMT-polyclonal antibody was able to bind to human prostatic, breast and colo-rectal carcinoma cell lines. MoAbs were then generated by standard procedures from animals hyperimmunized with high-titer CREF antisera coated CREF 4 NMT cells. As shown in Fig. 4, these MoAbs (MoAb 1.5.6 and MoAb 5.3.1.3) have good reactivity with LNCaP as well as other human carcinomas. In contrast, these MoAbs do not bind to HO-1, WI-38 or CREF cells. These results clearly indicate that the gene(s) which have been transferred to CREF-Trans 6 cells from LNCaP DNA are being expressed in transfected cells and they can be used to generate MoAbs which react with the original cells from which human tumor DNA was isolated.

The CREF-Trans 6 system has now been tested for the expression of tumor-inducing gene(s) from other human tumor cell lines as well as primary human tumor DNA samples. A tumorigenic phenotype from a human breast carcinoma cell line (T47D), a human glioblastoma multiform (stage IV astrocytoma) cell line (GBM-18) and primary human glioblastoma multiform (stage IV astrocytoma) DNA from a primary tumor have been successfully transferred. In the case of CREF-T47D transfectants, Alu sequences have been detected which, as expected, are different in size than those found in CREF 4 NMT cells. In addition, by using this antibody-coating technique with high specific activity CREF antisera, a MoAb (4.2.6) has been generated which binds to T47D as well as MCF-7 cells, but not to SW480, LNCaP, HO-1 or CREF cells (Fig. 5). A second MoAb (5.1.4) generated following exposure of animals to CREF-Trans 6 antisera coated CREF-T47D cells, not only binds to T47D and MCF-7, but it also reacts with LNCaP cells. These results suggest that the epitope recognized by MoAb 5.1.4 is expressed in breast and prostatic carcinoma cells, but not to the same degree in SW480 or HO-1 cells. Further studies of MoAbs 5.1.4, indicate that this MoAb retains good specificity for human breast carcinoma cells following subcloning and ascites formation (Fig. 6). A further analysis of these MoAbs (as well as MoAbs 1.5.6 and 5.3.1.3 generated against CREF 4 NMT cells) with a larger number of cell types, as well an analysis of sectioned tumor tissue, will determine the specificity and diagnostic utility of these MoAbs.

In summary, a general method has been developed for identifying genes and producing immunological reagents which encode TAA of human origin. An association between the tumor-inducing gene transferred by transfection into CREF cells and human encoded gene products (TAAs) was suggested by the ability to generate both polyclonal and MoAbs which bind to human prostatic carcinoma cells. In addition, by using the CREF/Transfection/Monoclonal Antibody technology gene(s) which may mediate or are associated with human breast carcinoma and glioblastoma multiform have been transferred and both polyclonal and MoAbs have been developed which recognize antigens expressed in human breast carcinomas and other carcinomas. The CREF/Transfection/Monoclonal Antibody technology has also been used to identify defined transfected and expressed surface molecules, including the cloned human 170,000 molecular weight (P-glycoprotein) multi-drug resistance gene. CREF and human tumor (breast carcinoma and glioblastoma) cells were transfected with the cloned mdr-1 gene, selected for colchicine resistance and the presence and expression of the mdr-1 gene was demonstrated by Southern and Northern analysis, respectively. These transfected CREF cells were then used to generate MoAbs which react with the P-glycoprotein expressed in transfected human breast and glioblastoma cells expressing an MDR phenotype.

### References

1. Babiss, L.E., et al. (1985) Science 228:1099.
2. Duigou, G.J, et al. (1990) Molecular and Cellular Biology 10:2027.
3. Greiner, J.W., et al. (1987) Science 235:895.
4. Leon, J.A., et al. (1989) Anticancer Research 9:1639.
5. Guarini, L., et al. (1990) Intl. J. of Cancer, in press.
6. Silverbert, E. and Lubera, J.A.(1989) CA Cancer J. Clin. 39:3.
7. Coffey, D.S., et al. (Eds.) (1987) Current Concepts and Approaches to the Study of Prostate Cancer, Allan R. Liss, Inc. New York.
8. Rijnders, A.W.M., et al. (1985) Biochem. Biophys. Res. Commun. 132:548.
9. Peehl, D.M., et al. (1987) The Prostate 10:281.
10. Buttyan, R., et al. (1987) The Prostate 11:327.
11. Cooper, C.S., et al. (1984) Nature 311:29.
12. Brown, B., et al. (1984) Carcenogenesis 5:1323.
13. Young, D., et al. (1986) Cell 45:711.
14. Fisher, P.B., et al. (1982) Proc Natl Acad Sci USA 79:3527.
15. Ashburner, M. and Ronner, J.J. (1979) Cell 17:241.
16. Levinson, W., et al. (1980) Biochem. Biophys. Acta 606:170.
17. Anathan, J., et al. (1986) Science 232:522.
18. Buttyan, R., et al. (1986) The Gerontologist 26:73A.
19. Fidler, I.J. and Hart, I.R. (1982) Science 217:998.
20. Poste, G. and Greig, R. (1982) Invasion and Metatstasis 2:137.
21. Heppner, G.H. (1984) Cancer Res. 44:2259.
22. Babiss, L.E., et al. (1984) J. Virol. 49:731.
23. Jelinek, W.R., et al. (1980) Proc Natl Acad Sci USA 77:1398.
24. Chao, M.V., et al. (1986) Science 232:518.
25. Kaiser, K. and Murray, N. DNA Cloning: A Practical Approach, vol. 1, Glover D.M. (Ed) (1985).
26. Benton, W.D. and Davis, R.W. (1977) Science 196:180.
27. Favalaro, J., et al. (1980) Methods Enzymol. 65:718.
28. Maniatis, T., et al. Molecular Cloning: A Laboratory Manual, Cold Spring Harbor Laboratory.
29. St John, T.P. and Davis, R.W. (1979) Cell 16:443.
30. Mangiarotti, G., et al. (1981) Nucleic Acids Res. 9:947.
31. Zuker, C. and Lodish H.F. (1981) Proc Natl Acad Sci USA 78:5386.
32. Scott, M.R.D., et al. (1983) Cell 34:557.
33. Maxim, A.M. and Gilbert, W. (1977) Proc Natl Acad Sci USA 34:360.
34. Okayama, H. and Berg, P. (1985) Mol. Cell. Biol. 5:1136.
35. Mulligan, R.C. and Berg, P.L. (1981) Proc Natl Acad Sci USA 78:2972.
36. Liaw, W.S., Duigou G.J., Ng A.K. and Fisher P.B., unpublished data, 1987.
37. Dorsch-Hasler K., et al. (1980) J. Virol. 34:385.
38. Greiner, J.W., et al. (1985) Pharmacol. Therap. 32:209.
39. Drebin, J.A., et al. (1985) Nature 312:545.
40. Roth, J.A., et al. (1984) Surgery 96:264.
41. Roth, J.A., et al. (1986) J. Immunol. 137:2385.
42. Hollingsworth, N.A., et al. (1986) Cancer Res. 46:2482.
43. Brickell, P.M., et al. (1983) Nature 306:756.
44. Carpenter, C.D., et al. (1984) Cell 36:663.
45. Fasano, O., et al. (1985) Mol. Cell Biol.
46. Liaw, W.S., et al. (1987) Amer. Urol. Assoc., Abstract.
47. Babiss L.E., et al. (1983) J. Virol. 46:454.
48. Weinberg, R.A. (1985) Science 230:770.
49. Bishop, J.M. (1983) Ann. Rev, Biochem. 52:301.
50. Schlom, J., et al. In: Klein, G., Weinhouse, S. and Light, S. (Eds), Advances in Cancer Research, Academic Press, New York, 43:143 (1985).
51. Reisfeld, R.A. and Ferrone, S. (Eds): Melanoma Antigens and Antibodies, Plenum Press, New York, 1982.
52. Liu, Z., Spooner, R.J.R., Fisher, P.B. and Ng, A.K., unpublished data, 1986.
53. Liu, Z., Borek, C. and Ng, A.K., unpublished data, 1987.
54. Kohler, G. and Milstein, C. (1975) Nature 256:495.
55. Nussenzweig, M.C., et al. (1982) Proc Natl Acad Sci USA 79:161.
56. Ng, A.K., et al. (1981) J. Immunol. 127:443.
57. Giacomini, P., et al. (1984) J. Immunol. 188:1649.
58. O'Farrell, J. (1975) J. Biol. Chem. 250:4007.
59. Garrets, J.I. (1979) J. Biol. Chem. 254:7951.
60. Bishop, J.M.:(1985) Cell 42:23.
61. Der, C.J., et al. (1982) Proc Natl Acad Sci USA 79:3637.
62. Goldfarb, M., et al. (1982) Nature 296:404.
63. Parada, L., et al. (1982) Nature 297:474.
64. Bookstein, R. et al. (1990) Science 242:1563.
65. Bookstein, R., et al. (1990) Proc Natl Acad Sci USA 87:7762.
66. Konig, J.J., et al. (1988) Cancer Genet. Cytogenet. 34:91.
67. Brothman, A.R. (1990) Cancer Res. 50:3795.
68. Carter, B.S., et al. (1990) Proc Natl Acad Sci USA 87:8751.
69. Blair, D.G., et al. (1982) Science 281:1122.
70. Fasano, O., et al. (1984) Mol. Cell Biol. 4:1695.
71. Yuasa, Y., et al. (1990) Oncogene 5:589.
72. Frischauf, A.M., et al. (1983) J. Mol. Biol. 170:827.
73. Benton, W.D. and Davis, R. (1977) Science 196:180.
74. Bovi, P.D. and Basilico, C. (1987) Proc Natl Acad Sci USA 84:5660.
75. Higashi, T, et al. (1990) Proc Natl Acad Sci USA 84:2409.
76. Feinberg A & Vogelstein B (1983) Anal. Biochem. 132:6.
77. Reddy P.G., et al. (1988) Proc Natl Acad Sci USA 85:9081.
78. Reddy P.G., et al. (1989) Gene 76:145.
79. Nelson, D.L., et al. (1989) Proc Natl Acad Sci USA 86:6686.
80. Burke, D.T., et al. (1987) Science 236:806.
81. Jelinek, W.R., et al. (1982) Ann. Rev. Biochem. 51:813.
82. Triglia, T., et al. (1988) Nucleic Acids Res. 16:8186.
83. Gubler U and Hoffman B.J. (1984) Proc Natl Acad Sci USA 81:2035.
84. Sargent, T.D. and Dawid, I.B. (1983) Science 222:135.
85. Davanco, P., et al. (1984) Proc Natl Acad Sci USA 81:2035.
86. Timberlake, W.E. (1980) Devel. Biol. 78:497.
87. Wieland, I., et al. (1990) Proc Natl Acad Sci USA 87:2720.
88. Young, R.A. and Davis, R.W. (1983) Proc Natl Acad Sci USA 80:1194.
89. Isaacs, J.T. (1987) In: Coffey, D.S., et al. (Eds.) Current Concepts and Approaches to the Study of Prostate Cancer, pp. 513-575, Allan R. Liss, Inc., NY.
90. Bruce, J.N., et al. (1989) Cancer Cells, Vol. 7, Molecular Diagnostics of Human Cancer, pp. 363-370, Cold Spring Harbor Lab, NY.
91. Simmons, D.M. et al. (1989) J. Histotech 12:169.
92. Thompson, T.C., et al. (1989) Cell 56:917.
93. Su, Z.Z., et al. (1990) Mol. Carcinogenesis, in press.
94. Fisher, P.B., et al. (1979) Cancer Res. 39:3051.
95. Boylan, J.F., et al. (1990) Anticancer Res. 10:717.
96. Scuder P., et al. (1985) Med. Oncol. & Tumor Pharmacother. 2:233.
97. Hollingsworth, M.A. et al. (1986) Cancer Res. 46:2482.
98. Guarini, L., et al. (1989) Cancer Immunol. Immunother. 30:262.
99. Goldstein, N.I., (1990) Anticancer Res., in press.
100. Guarini, L., et al. (1990) Int. J. Cancer 46, in press.
101. Chu, T.M., (1986) Urology XXVI:487.
102. Goldenberg, D.M. (Ed) (1990) Cancer Res. 50 Supp:774s.

## Claims

1. A method for preparing a hybridoma cell line which produces a monoclonal antibody which specifically recognizes and binds to a tumor associated antigen associated with a neoplastic, human cell which comprises:
a) co-transfecting the CREF-Trans 6 cell line (ATCC Accession No. CRL 10584), with DNA isolated from a neoplastic, human cell and a plasmid, which encodes a selectable or identifiable trait encoding resistance to an antibiotic;
b) selecting transfected cells which express the selectable or identifiable trait;
c) recovering the transfected cells so selected;
d) injecting the transfected cells so recovered into a suitable first murine host;
e) maintaining the resulting first murine host for a period of time effective to induce the injected transfected cells to form a tumor in the first murine host;
f) isolating the resulting tumor from the first murine host;
g) obtaining tumor cells from the tumor so isolated;
h) coating the tumor cells so obtained with an antiserum generated against the CREF-Trans 6 cell line;
i) injecting the antiserum-coated cells into the suitable nonhuman second hosts;
j) screening the resulting second hosts to identify hosts which produce serum reactive with the neoplastic, human cell;
k) removing spleens from the second hosts so identified;
l) preparing from the spleens so removed hybridomas; and
m) recovering therefrom a hybridoma cell line which produces an antibody which specifically recognizes and binds to the cell surface antigen.

2. The method of claim 1, wherein the neoplastic, human cell is a benign cell, a metastatic cell, a human prostatic carcinoma cell derived from cell line LNCaP, a human breast carcinoma cell derived from cell line T47D, a human colorectal carcinoma cell derived from cell line SW480, a human glioblastoma multiform (Stage IV astrocytoma) cell derived from cell line GBM-18 or a human glioblastoma multiform (Stage IV astrocytoma) cell derived from a primary tumor.

3. The method of any one of claims 1 to 2, wherein the suitable second host is a murine host or a non-human primate host.

4. The method of any of claims 1 to 3, wherein the cell surface antigen is a tumor associated antigen, a growth factor receptor, a viral-encoded, surface-expressed antigen, encoded by an oncogene product, a surface epitope, a membrane protein which mediates classical multi-drug resistance, a membrane protein which mediates atypical multi-drug resistance, an antigen which mediates a tumorigenic phenotype, an antigen which mediates a metastatic phenotype, an antigen which suppresses a tumorigenic phenotype, an antigen which suppresses a metastatic phenotype, an antigen which is recognized by a specific immunological effector cell or an antigen which is recognized by a non-specific immunological effector cell.

5. The method of claim 4, wherein the specific immunological effector cell is a T-cell.

6. The method of claim 4, wherein the non-specific immunological effector cell is a macrophage cell or a natural killer cell.

7. A method producing a monoclonal antibody which specifically recognizes and binds to a cell surface antigen associated with a neoplastic, human cell which comprises:
(a) producing a hybridoma according to the methods of any one of claims 1-6; and
(b) recovering from the hybridoma so produced the monoclonal antibody.

8. A method for preparing a polyclonal antibody which specifically recognizes and binds to a cell surface antigen associated with a neoplastic, human cell which comprises:
a) co-transfecting the CREF Trans-6 cell line with DNA isolated from a neoplastic human cell and DNA encoding a selectable or identifiable trait;
b) selecting transfected cells which express the selectable or identifiable trait;
c) recovering the transfected cells so selected;
d) injecting the transfected cells so recovered into a suitable first murine host;
e) maintaining the resulting first murine host for a period of time effective to induce the injected transfected cells to form a tumor in the first murine host;
f) isolating the resulting tumor from the first murine host;
g) obtaining tumor cells from the tumor so isolated; and
h) coating the tumor cells so obtained with an antiserum generated against the CREF-Trans 6 cell line;
i) injecting the antiserum-coated cells into the suitable nonhuman second hosts;
j) screening the resulting second hosts to identify hosts which produce serum reactive with the neoplastic, human cell;
k) recovering from the second hosts so identified the polyclonal antibody.

9. The method of claim 8, wherein the neoplastic, human cell is a benign cell, a metastatic cell, a human prostatic carcinoma cell derived from cell line LNCaP, a human breast carcinoma cell derived from cell line T47D, a human colorectal carcinoma cell derived from cell line SW480, a human glioblastoma multiform (Stage IV astrocytoma) cell derived from cell line GBM-18 or a human glioblastoma multiform (Stage IV astrocytoma) cell derived from a primary tumor.

10. The method of any one of claims 8 or 9, wherein the DNA encoding the selectable or identifiable trait is plasmid DNA encoding resistance to an antibiotic.

11. The method of claim 10, wherein the plasmid DNA comprises pSV2-Neo.

12. The method of claim 11, wherein the antibiotic is G418.

13. The method of any one of claims 8 to 11, wherein the suitable second host is a murine host or a non-human primate host.

14. The method of any one of claims 8 to 11, wherein the cell surface antigen is a tumor associated antigen, a growth factor receptor, a viral-encoded, surface-expressed antigen, encoded by an oncogene, a membrane protein which mediates classical multi-drug resistance, a membrane protein which mediates atypical multi-drug resistance, an antigen which mediates a tumorigenic phenotype, an antigen which mediates a metastatic phenotype, an antigen which suppresses a tumorigenic phenotype, an antigen which suppresses a metastatic phenotype, an antigen which is recognized by a specific immunological effector cell or an antigen which is recognized by a non-specific immunological effector cell.

15. The method of claim 14, wherein the specific immunological effector cell is a T-cell.

16. The method of claim 14, wherein the non-specific immunological effector cell is a macrophage cell or a natural killer cell.

17. A method of preparing DNA encoding a cell surface antigen associated with a neoplastic, human cell which comprises:
a) co-transfecting the CREF-Trans 6 cell line with DNA isolated from a neoplastic, human cell and DNA encoding a selectable or identifiable trait;
b) selecting transfected cells which express the selectable or identifiable trait;
c) recovering the transfected cells so selected;
d) injecting the transfected cells so recovered into a suitable first murine host;
e) maintaining the resulting first murine host for a period of time effective to induce the injected transfected cells to form a tumor in the first murine host;
f) isolating the resulting tumor from the first murine host;
g) obtaining tumor cells from the tumor so isolated; and
h) recovering DNA encoding the cell surface antigen associated with the neoplastic human cell from the tumor cells so obtained.

18. The method of claim 17, wherein the neoplastic, human cell is a benign cell, a metastic cell, a human prostatic carcinoma cell derived from cell line LNCaP, a human breast carcinoma cell derived from cell line T47D, a human colorectal carcinoma cell derived from cell line SW480, a human glioblastoma multiform (Stage IV astrocytoma) cell derived from cell line GBM-18 or a human glioblastoma multiform (Stage IV astrocytoma) cell derived from a primary tumor.

19. The method of claim 17 to 18, wherein the DNA encoding the selectable or identifiable trait is plasmid DNA encoding resistance to an antibiotic.

20. The method of claim 19, wherein the plasmid DNA comprises pSV2-Neo.

21. The method of claim 20, wherein the antibiotic is G418.

22. The method of any one of claims 17 to 21, wherein the cell surface antigen is a tumor associated antigen, a growth factor receptor, a viral-encoded, surface-expressed antigen, encoded by an oncogene product, a surface epitope, a membrane protein which mediates classical multi-drug resistance, a membrane protein which mediates atypical multi-drug resistance, an antigen which mediates a tumorigenic phenotype, an antigen which mediates a metastatic phenotype, an antigen which suppresses a tumorigenic phenotype, an antigen which suppresses a metastatic phenotype, an antigen which is recognized by a specific immunological effector cell or an antigen which is recognized by a non-specific immunological effector cell.

23. The method of claim 22, wherein the specific immunological effector cell is a T-cell.

24. The method of claim 22, wherein the non-specific immunological effector cell is a macrophage cell or a natural killer cell.

## Patentansprüche

1. Verfahren zur Herstellung einer Hybridom-Zelllinie, die einen monoclonalen Antikörper produziert, welcher ein tumorassoziiertes Antigen, das mit einer neoplastischen, menschlichen Zelle assoziiert ist, spezifisch erkennt und bindet, umfassend:
(a) Cotransfizieren der CREF-Trans 6-Zelllinie (ATCC-Hinterlegungs-Nr. CRL 10584), mit DNA, isoliert aus einer neoplastischen, menschlichen Zelle, und einem Plasmid, welches ein selektierbares oder identifizierbares Merkmal codiert, das eine Resistenz für ein Antibiotikum codiert;
(b) Selektieren transfizierter Zellen, die das selektierbare oder identifizierbare Merkmal exprimieren;
(c) Gewinnung der so selektierten transfizierten Zellen;
(d) Injizieren der transfizierten und so gewonnenen Zellen in einen geeigneten ersten murinen Wirt;
(e) Halten des hergestellten ersten murinen Wirts für einen Zeitraum, der ausreicht, um die injizierten transfizierten Zellen zu veranlassen, einen Tumor in dem ersten murinen Wirt zu bilden;
(f) Isolieren des entstandene Tumors vom ersten murinen Wirt;
(g) Gewinnung von Tumorzellen aus dem so isolierten Tumor;
(h) Beschichten der so erhaltenen Tumorzellen mit einem Antiserum, hergestellt gegen die CREF-Trans 6-Zelllinie;
(i) Injizieren der Antiserum-beschichteten Zellen in die geeigneten nichtmenschlichen zweiten Wirte;
(j) Durchmusterung der erhaltenen zweiten Wirte zur Identifizierung von Wirten, welche ein Serum produzieren, welches mit einer neoplastischen, menschlichen Zelle reagiert;
(k) Entfernen der Milzen von den so identifizierten zweiten Wirten;
(l) Herstellen von Hybridomen aus den so entfernten Milzen; und
(m) Gewinnen einer Hybridom-Zelllinie, welche einen Antikörper produziert, der das Zelloberflächenantigen spezifisch erkennt und bindet.

2. Verfahren nach Anspruch 1, wobei die neoplastische, menschliche Zelle eine gutartige Zelle, eine metastatische Zelle, eine menschliche Prostatakarzinomzelle gewonnen aus der Zelllinie LNCaP, eine menschliche Brustkarzinomzelle gewonnen aus der Zelllinie T47D, eine menschliche colorectale Karzinomzelle gewonnen aus der Zelllinie SW480, eine menschliche multiforme Glioblastom (Stufe IV-Astrocytom)-Zelle gewonnen aus der Zelllinie GBM-18 oder eine menschliche multiforme Glioblastom (Stufe IV-Astrocytom)-Zelle gewonnen aus einem Primärtumor ist.

3. Verfahren nach einem der Ansprüche 1 bis 2, wobei der geeignete zweite Wirt ein muriner Wirt oder ein nicht-menschlicher Primat ist.

4. Verfahren nach einem der Ansprüche 1 bis 3, wobei das Zelloberflächenantigen ein tumorassoziiertes Antigen, ein Rezeptor für einen Wachstumsfaktor, ein viral-codiertes, oberflächenexprimiertes Antigen, codiert von einem onkogenen Produkt, ein Oberflächenepitop, ein Membranprotein, welches klassische Mehrfacharzneimittelresistenz überträgt, ein Membranprotein, welches atypische Mehrfacharzneimittelresistenz überträgt, ein Antigen, welches einen tumorerzeugenden Phänotyp überträgt, ein Antigen, welches einen metastatischen Phänotyp überträgt, ein Antigen, welches einen tumorerzeugenden Phänotyp unterdrückt, ein Antigen, welches einen metastatischen Phänotyp unterdrückt, ein Antigen, welches durch eine spezifische immunologische Effektorzelle erkannt wird, oder ein Antigen ist, welches durch eine nicht-spezifische immunologische Effektorzelle erkannt wird.

5. Verfahren nach Anspruch 4, wobei die spezifische immunologische Effektorzelle eine T-Zelle ist.

6. Verfahren nach Anspruch 4, wobei die nicht-spezifische immunologische Effektorzelle eine Makrophagen-Zelle oder eine natürliche Killerzelle ist.

7. Verfahren zur Herstellung eines monoclonalen Antikörpers, der ein Zelloberflächenantigen, welches assoziiert ist mit einer neoplastischen, menschlichen Zelle, spezifisch erkennt und bindet, umfassend:
(a) Herstellung eines Hybridoms gemäß dem Verfahren nach einem der Ansprüche 1 bis 6; und
(b) Gewinnen des monoclonalen Antikörpers von dem so hergestellten Hybridom.

8. Verfahren zur Herstellung eines polyclonalen Antikörpers, der ein Zelloberflächenantigen, welches assoziiert ist mit einer neoplastischen, menschlichen Zelle, spezifisch erkennt und bindet, umfassend:
(a) Cotransfizieren der CREF-Trans 6-Zelllinie mit DNA, isoliert aus einer neoplastischen, menschlichen Zelle, und DNA, die ein selektierbares oder identifizierbares Merkmal codiert;
(b) Selektieren transfizierter Zellen, die das selektierbare oder identifizierbare Merkmal exprimieren;
(c) Gewinnung der so selektierten, transfizierten Zellen;
(d) Injizieren der so gewonnenen transfizierten Zellen in einen geeigneten ersten murinen Wirt;
(e) Halten des hergestellten ersten murinen Wirts für einen Zeitraum, der ausreicht, um die injizierten transfizierten Zellen zu veranlassen, einen Tumor in dem ersten murinen Wirt zu bilden;
(f) Isolieren des entstandenen Tumors vom ersten murinen Wirt;
(g) Gewinnung von Tumorzellen aus dem so isolierten Tumor; und
(h) Beschichten der so erhaltenen Tumorzellen mit einem Antiserum, erzeugt gegen die CREF-Trans 6-Zelllinie;
(i) Injizieren der Antiserum-beschichteten Zellen in die geeigneten nichtmenschlichen zweiten Wirte;
(j) Durchmusterung der erhaltenen zweiten Wirte zur Identifizierung von Wirten, welche ein Serum produzieren, welches mit einer neoplastischen, menschlichen Zelle reagiert;
(k) Gewinnung des polyclonalen Antikörpers von den so identifizierten zweiten Wirten.

9. Verfahren nach Anspruch 8, wobei die neoplastische, menschliche Zelle eine gutartige Zelle, eine metastatische Zelle, eine menschliche Prostatakarzinomzelle gewonnen aus der Zelllinie LNCaP, eine menschliche Brustkarzinomzelle gewonnen aus der Zelllinie T47D, eine menschliche colorectale Karzinomzelle gewonnen aus der Zelllinie SW480, eine menschliche multiforme Glioblastom (Stufe IV-Astrocytom)-Zelle gewonnen aus der Zelllinie GBM-18 oder eine menschliche multiforme Glioblastom (Stufe IV-Astrocytom)-Zelle gewonnen aus einem Primärtumor ist.

10. Verfahren nach einem der Ansprüche 8 oder 9, wobei die DNA, die das selektierbare oder identifizierbare Merkmal codiert, Plasmid-DNA ist, die für eine Resistenz gegen ein Antibiotikum codiert.

11. Verfahren nach Anspruch 10, wobei die Plasmid-DNA pSV2-Neo umfasst.

12. Verfahren nach Anspruch 11, wobei das Antibiotikum G418 ist.

13. Verfahren nach einem der Ansprüche 8 bis 11, wobei der geeignete zweite Wirt ein muriner Wirt oder ein nicht-menschlicher Primat ist.

14. Verfahren nach einem der Ansprüche 8 bis 11, wobei das Zelloberflächenantigen ein tumorassoziiertes Antigen, ein Rezeptor für einen Wachstumsfaktor, ein viral-codiertes, oberflächenexprimiertes Antigen, codiert von einem Onkogen, ein Membranprotein, welches klassische Mehrfacharzneimittelresistenz überträgt, ein Membranprotein, welches atypische Mehrfacharzneimittelresistenz überträgt, ein Antigen, welches einen tumorerzeugenden Phänotyp überträgt, ein Antigen, welches einen metastatischen Phänotyp überträgt, ein Antigen, welches einen tumorerzeugenden Phänotyp unterdrückt, ein Antigen, welches einen metastatischen Phänotyp unterdrückt, ein Antigen, welches durch eine spezifische immunologische Effektorzelle erkannt wird, oder ein Antigen ist, welches durch eine nicht-spezifische immunologische Effektorzelle erkannt wird.

15. Verfahren nach Anspruch 14, wobei die spezifische immunologische Effektorzelle eine T-Zelle ist.

16. Verfahren nach Anspruch 14, wobei die nicht-spezifische immunologische Effektorzelle eine Makrophagen-Zelle oder eine natürliche Killerzelle ist.

17. Verfahren zur Herstellung von DNA, die ein Zelloberflächenantigen codiert, welches assoziiert ist mit einer neoplastischen, menschlichen Zelle, umfassend:
(a) Cotransfizieren der CREF-Trans 6-Zelllinie mit DNA, die von einer neoplastischen, menschlichen Zelle isoliert wurde, und DNA, die ein selektierbares oder identifizierbares Merkmal codiert;
(b) Selektieren transfizierter Zellen, die das selektierbare oder identifizierbare Merkmal exprimieren;
(c) Gewinnung der so selektierten transfizierten Zellen;
(d) Injizieren der so gewonnenen transfizierten Zellen in einen geeigneten ersten murinen Wirt;
(e) Halten des hergestellten ersten murinen Wirts für einen Zeitraum, der ausreicht, um die injizierten transfizierten Zellen zu veranlassen, einen Tumor in dem ersten murinen Wirt zu bilden;
(f) Isolieren des entstandene Tumors vom ersten murinen Wirt;
(g) Gewinnung von Tumorzellen aus dem so isolierten Tumor; und
(h) Gewinnung von DNA, die das Zelloberflächen-Antigen codiert, welches der mit neoplastischen, menschlichen Zelle assoziiert ist, von den so erhaltenen Tumorzellen.

18. Verfahren nach Anspruch 17, wobei die neoplastische, menschliche Zelle eine gutartige Zelle, eine metastatische Zelle, eine menschliche Prostatakarzinomzelle gewonnen aus der Zelllinie LNCaP, eine menschliche Brustkarzinomzelle gewonnen aus der Zelllinie T47D, eine menschliche colorectale Karzinomzelle gewonnen aus der Zelllinie SW480, eine menschliche multiforme Glioblastom (Stufe IV Astrocytom)-Zelle gewonnen aus der Zelllinie GBM-18 oder eine menschliche multiforme Glioblastom (Stufe IV-Astrocytom)-Zelle gewonnen aus einem Primärtumor ist.

19. Verfahren nach Anspruch 17 bis 18, wobei die DNA, die das selektierbare oder identifizierbare Merkmal codiert, Plasmid-DNA ist, die eine Resistenz für ein Antibiotikum codiert.

20. Verfahren nach Anspruch 19, wobei die Plasmid-DNA pSV2-Neo umfasst.

21. Verfahren nach Anspruch 20, wobei das Antibiotikum G418 ist.

22. Verfahren nach einem der Ansprüche 17 bis 21, wobei das Zelloberflächenantigen ein tumorassoziiertes Antigen, ein Rezeptor für einen Wachstumsfaktor, ein viral-codiertes, oberflächenexprimiertes Antigen, codiert von einem onkogenen Produkt, ein Oberflächenepitop, ein Membranprotein, welches klassische Mehrfacharzneimittelresistenz überträgt, ein Membranprotein, welches atypische Mehrfacharzneimittelresistenz überträgt, ein Antigen, welches einen tumorerzeugenden Phänotyp überträgt, ein Antigen, welches einen metastatischen Phänotyp überträgt, ein Antigen, welches einen tumorerzeugenden Phänotyp unterdrückt, ein Antigen, welches einen metastatischen Phänotyp unterdrückt, ein Antigen, welches durch eine spezifische immunologische Effektorzelle erkannt wird, oder ein Antigen ist, welches durch eine nicht-spezifische immunologische Effektorzelle erkannt wird.

23. Verfahren nach Anspruch 22, wobei die spezifische immunologische Effektorzelle eine T-Zelle ist.

24. Verfahren nach Anspruch 22, wobei die nicht-spezifische immunologische Effektorzelle eine Makrophagen-Zelle oder eine natürliche Killerzelle ist.

## Revendications

1. Procédé pour préparer une lignée cellulaire d'hybridome qui produit un anticorps monoclonal qui reconnaît spécifiquement et se lie à un antigène associé à une tumeur associé avec une cellule humaine néoplasique, comprenant :
a) la co-transfection de la lignée cellulaire CREF-Trans 6 (ATCC accès N° CRL 10584), avec de l'ADN isolé d'une cellule humaine néoplasique et un plasmide, qui code pour un trait sélectionnable et identifiable codant pour une résistance à un antibiotique ;
b) la sélection des cellules transfectées qui expriment le trait sélectionnable ou identifiable ;
c) la récupération des cellules transfectées ainsi sélectionnées ;
d) l'injection des cellules transfectées ainsi récupérées dans un premier hôte murin approprié ;
e) le maintien du premier hôte murin résultant pendant une période de temps efficace pour que les cellules transfectées injectées soient induites à former une tumeur dans le premier hôte murin ;
f) l'isolement de la tumeur résultante à partir du premier hôte murin ;
g) l'obtention de cellules tumorales à partir de la tumeur ainsi isolée ;
h) le recouvrement des cellules tumorales ainsi obtenues avec un antisérum généré contre la lignée cellulaire CREF-Trans 6 ;
i) l'injection des cellules recouvertes d'antisérum dans les seconds hôtes non humains appropriés ;
j) le criblage des seconds hôtes résultants pour identifier les hôtes qui produisent du sérum réactif avec la cellule humaine néoplasique ;
k) la récupération des rates des seconds hôtes ainsi identifiés ;
l) la préparation d'hybridomes à partir des rates ainsi récupérées ; et
m) la récupération à partir de là de lignée cellulaire d'hybridome, qui produit un anticorps qui reconnaît spécifiquement les cellules et se lie à l'antigène de surface cellulaire.

2. Procédé selon la revendication 1, dans lequel la cellule humaine néoplasique est une cellule bénigne, une cellule métastasique, une cellule de carcinome prostatique humain issue de la lignée cellulaire LNCaP, une cellule de carcinome du sein humain issue de la lignée cellulaire T47D, une cellule de carcinome colorectal humain issue de la lignée cellulaire SW480, une cellule multiforme de glioblastome humain (astrocytome de stade IV) issue de la lignée cellulaire GBM-18 ou une cellule multiforme de glioblastome humain (astrocytome de stade IV) issue d'une tumeur primaire.

3. Procédé selon l'une quelconque des revendications 1 à 2, dans lequel le second hôte approprié est un hôte murin ou un hôte primate non-humain.

4. Procédé selon l'une quelconque des revendications 1 à 3, dans lequel l'antigène de surface cellulaire est un antigène associé à une tumeur, un récepteur d'un facteur de croissance, un produit codé par un oncogène, un antigène exprimé en surface, codé par un virus, un épitope de surface, une protéine membranaire qui médie une résistance multiple aux drogues classique, une protéine membranaire qui médie une résistance multiple aux drogues atypique, un antigène qui médie un phénotype tumorigène, un antigène qui médie un phénotype métastasique, un antigène qui supprime un phénotype tumorigène, et un antigène qui supprime un phénotype métastasique, un antigène qui est reconnu par une cellule effectrice immunologique spécifique ou un antigène qui est reconnu par une cellule effectrice immunologique non-spécifique.

5. Procédé selon la revendication 4, dans lequel la cellule effectrice immunologique spécifique est une cellule T.

6. Procédé selon la revendication 4, dans lequel la cellule effectrice immunologique non-spécifique est une cellule de macrophage ou une cellule « natural killer ».

7. Procédé pour produire un anticorps monoclonal qui reconnaît spécifiquement un antigène de surface cellulaire associé à une cellule humaine néoplasique, qui comprend :
a) la production d'une hybridome selon les procédés de l'une quelconque des revendications 1-6 ; et
b) la récupération à partir de l'hybridome ainsi produit de l'anticorps monoclonal.

8. Procédé pour préparer un anticorps polyclonal qui reconnaît spécifiquement et se lie à un antigène de surface cellulaire associé à une cellule humaine néoplasique, qui comprend :
a) la co-transfection de la lignée cellulaire CREF-Trans 6, avec de l'ADN isolé d'une cellule humaine néoplasique ou un ADN codant pour un trait sélectionnable ou identifiable ;
b) la sélection des cellules transfectées qui expriment le trait sélectionnable ou identifiable ;
c) la récupération des cellules transfectées ainsi sélectionnées ;
d) l'injection des cellules transfectées ainsi récupérées dans un premier hôte murin approprié ;
e) le maintien du premier hôte murin résultant pendant une période de temps efficace pour que les cellules transfectées injectées soient induites à former une tumeur dans le premier hôte murin ;
f) l'isolement de la tumeur résultante à partir du premier hôte murin ;
g) l'obtention de cellules tumorales à partir de la tumeur ainsi isolée ;
h) le recouvrement des cellules tumorales ainsi obtenues avec un antisérum généré contre la lignée cellulaire CREF-Trans 6 ;
i) l'injection des cellules recouvertes d'antisérum dans les seconds hôtes non humains appropriés ;
j) le criblage des seconds hôtes résultants pour identifier les hôtes qui produisent du sérum réactif avec la cellule humaine néoplasique ;
k) la récupération à partir des seconds hôtes ainsi identifiés de l'anticorps polyclonal.

9. Procédé selon la revendication 8, dans lequel la cellule humaine néoplasique est une cellule bénigne, une cellule métastasique, une cellule de carcinome prostatique humain issue de la lignée cellulaire LNCaP, une cellule dé carcinome du sein humain issue de la lignée cellulaire T47D, une cellule de carcinome colorectal humain issue de la lignée cellulaire SW480, une cellule multiforme de glioblastome humain (astrocytome de stade IV) issue de la lignée cellulaire GBM-18 ou une cellule multiforme de glioblastome humain (astrocytome de stade IV) issue d'une tumeur primaire.

10. Procédé selon l'une quelconque des revendications 8 ou 9, dans lequel l'ADN codant pour le trait sélectionnable ou identifiable est un ADN plasmidique codant pour une résistance à un antibiotique.

11. Procédé selon la revendication 10, dans lequel l'ADN plasmidique comprend pSV2-Neo.

12. Procédé selon la revendication 11, dans lequel l'antibiotique est G418.

13. Procédé selon l'une quelconque des revendications 8 à 11, dans lequel le second hôte approprié est un hôte murin ou un hôte primate non-humain.

14. Procédé selon l'une des revendications 8 à 11, dans lequel l'antigène de surface cellulaire est un antigène associé à une tumeur, un récepteur d'un facteur de croissance, un produit codé par un oncogène, un antigène exprimé en surface, codé par un virus, un épitope de surface, une protéine membranaire qui médie une résistance multiple aux drogues classique, une protéine membranaire qui médie une résistance multiple aux drogues atypique, un antigène qui médie un phénotype tumorigène, un antigène qui médie un phénotype métastasique, un antigène qui supprime un phénotype tumorigène, et un antigène qui supprime un phénotype métastasique, un antigène qui est reconnu par une cellule effectrice immunologique spécifique ou un antigène qui est reconnu par une cellule effectrice immunologique non-spécifique.

15. Procédé selon la revendication 14, dans lequel la cellule effectrice immunologique spécifique est une cellule T.

16. Procédé selon la revendication 14, dans lequel la cellule effectrice immunologique non-spécifique est une cellule de macrophage ou une cellule « natural killer ».

17. Procédé pour préparer l'ADN codant pour un antigène de surface cellulaire associé à une cellule humaine, néoplasique qui comprend :
a) la co-transfection de la lignée cellulaire CREF-Trans 6, avec de l'ADN isolé d'une cellule humaine néoplasique ou un ADN codant pour un trait sélectionnable ou identifiable ;
b) la sélection des cellules transfectées qui expriment le trait sélectionnable ou identifiable ;
c) la récupération des cellules transfectées ainsi sélectionnées ;
d) l'injection des cellules transfectées ainsi récupérées dans un premier hôte murin approprié ;
e) le maintien du premier hôte murin résultant pendant une période de temps efficace pour que les cellules transfectées injectées soient induites à former une tumeur dans le premier hôte murin ;
f) l'isolement de la tumeur résultante à partir du premier hôte murin ;
g) l'obtention de cellules tumorales à partir de la tumeur ainsi isolée ; et
h) la récupération de l'ADN codant pour l'antigène de surface cellulaire associé à une cellule humaine néoplasique à partir de cellules tumorales ainsi obtenues.

18. Procédé selon la revendication 17, dans lequel la cellule humaine néoplasique est une cellule bénigne, une cellule métastasique, une cellule de carcinome prostatique humain issue de la lignée cellulaire LNCaP, une cellule de carcinome du sein humain issue de la lignée cellulaire T47D, une cellule de carcinome colorectal humain issue de la lignée cellulaire SW480, une cellule multiforme de glioblastome humain (astrocytome de stade IV) issue de la lignée cellulaire GBM-18 ou une cellule multiforme de glioblastome humain (astrocytome de stade IV) issue d'une tumeur primaire.

19. Procédé selon les revendications 17 à 18, dans lequel l'ADN codant pour le trait sélectionnable ou identifiable est un ADN plasmidique codant pour une résistance à un antibiotique.

20. Procédé selon la revendication 19, dans lequel l'ADN plasmidique comprend pSV2-Neo.

21. Procédé selon la revendication 20, dans lequel l'antibiotique est G418.

22. Procédé selon l'une quelconque des revendications 17 à 21, dans lequel l'antigène de surface cellulaire est un antigène associé à une tumeur, un récepteur d'un facteur de croissance, un produit codé par un oncogène, un antigène exprimé en surface, codé par un virus, un épitope de surface, une protéine membranaire qui médie une résistance multiple aux drogues classique, une protéine membranaire qui médie une résistance multiple aux drogues atypique, un antigène qui médie un phénotype tumorigène, un antigène qui médie un phénotype métastasique, un antigène qui supprime un phénotype tumorigène, et un antigène qui supprime un phénotype métastasique, un antigène qui est reconnu par une cellule effectrice immunologique spécifique ou un antigène qui est reconnu par une cellule effectrice immunologique non-spécifique.

23. Procédé selon la revendication 22, dans lequel l'effecteur immunologique spécifique est une cellule T.

24. Procédé selon la revendication 22, dans lequel la cellule effectrice immunologique non-spécifique est une cellule de macrophage ou une cellule « natural killer ».
